(19) Europäisches Patentamt
European Patent Office
Office européen des brevets

(11) Publication number: 0 383 205
A1

(12) # EUROPEAN PATENT APPLICATION

(21) Application number: 90102552.8

(22) Date of filing: 09.02.90

(51) Int. Cl.[5]: C07K 15/06, C12N 15/12, C12P 21/02, A61K 37/02, C12Q 1/68, A61K 37/36, //(A61K37/36,37:02)

(30) Priority: 17.02.89 US 310725

(43) Date of publication of application: 22.08.90 Bulletin 90/34

(84) Designated Contracting States: AT BE CH DE DK ES FR GB GR IT LI LU NL SE

(71) Applicant: AMERICAN CYANAMID COMPANY
1937 West Main Street P.O. Box 60
Stamford Connecticut 06904-0060(US)

(72) Inventor: Logan, John Steele
347B Old York Road
Robbinsville, New Jersey 08691(US)
Inventor: Baumbach, William Robert
32 Louellen Street
Hopewell, New Jersey 08525(US)

(74) Representative: Wächtershäuser, Günter, Dr.
Tal 29
D-8000 München 2(DE)

(54) **Somatotropin binding protein.**

(57) The present invention relates to the novel protein and DNA of serum somatotropin binding protein. The novel protein of the present invention uniquely contains a 17 amino acid "tail" on the carboxy terminal of the protein, produced by alternative splicing of RNA for the somatotropin receptor. The present invention also includes a method for identifying the serum somatotropin binding protein separate from the somatotropin receptor protein. This novel protein is useful in altering and affecting growth characteristics of animals.



Xerox Copy Centre

# SOMATOTROPIN BINDING PROTEIN

## Background of the Invention

The present invention relates to novel somatotropin binding proteins present in sera of animals and methods for identifying and differentiating proteins of the invention from somatotropin receptor proteins. The binding proteins of animals are not found in the isolated form of the present invention since they "bind" somatotropin in vivo.

The novel proteins of the present invention are useful in regulating growth of animals, including all animals having a somatotropin. For instance, the proteins of the present invention are useful in regulating growth of such animals as bovine, porcine, caprine, ovine, fish, avian (chicken, turkey, geese, etc.) and rabbit, amongst others. Further, other biological uses of the present proteins are directed toward specific regulation and/or modification of the activity of somatotropins, such as milk production, egg quality, wool growth and/or quality, etc. In human beings, the proteins of the present invention are useful in modifying the action of human somatotropin; for instance, regulating metabolism, wound healing, diabetes control and other human somatotropin mediated biological activities.

More specifically, antibodies raised against the somatotropin binding protein result in enhanced growth, a finding discussed in more detail herein.

Although the presence of a type of somatotropin binding protein in serum has been speculated, the sequence and molecular basis for the mechanism of production of this protein has not been known. For instance, Laron dwarfs defective in the production of functional somatotropin receptors are also defective in the production of functional somatotropin binding proteins in serum (Baumann et. al. 1987; Daughaday et. al. 1987). This suggests a close genetic relationship between the two proteins. Monoclonal and polyclonal antibodies binding to this somatotropin receptor also bind to the somatotropin binding protein in serum (Barnard and Waters, 1986; Baumann and Shaw, 1988). Purification of the somatotropin binding protein reveals that both the somatotropin receptor and the serum somatotropin binding protein have similar amino terminal sequences, and peptide mapping in rabbit suggests colinearity and identity between the extracellular domains of the somatotropin receptor and somatotropin binding protein (Leung et. al. 1987; Spencer et. al., 1988). Suggestions in the literature state that the somatotropin binding protein is derived from the somatotropin receptor by proteolytic cleavage (Leung et. al., 1987; Spencer et. al., 1988; Trivedi and Daughaday, 1988).

A recently published paper (Smith et. al., 1988) indicates that two forms of the mouse somatotropin receptor exist at the mRNA level and suggests that one form may be the mouse serum somatotropin binding protein. However, the sequence of the binding protein is not shown, and no evidence is presented to indicate the nature of the relationship between the mouse somatotropin receptor and the mouse serum somatotropin binding protein. The present invention, therefore, provides the concrete existence, identification, characterization and method to differentiate between the serum somatotropin binding protein and somatotropin receptor. The serum somatotropin binding protein has at least the additional 17 amino acid carboxy tail or equivalents thereof. Mouse has a 27 amino acid carboxy terminal chain. Equivalent tail lengths for other species may be longer or somewhat shorter in length, but functionally perform the same in vivo and in vitro.

It is the object of the present invention to provide novel proteins wherein the entire primary amino acid sequence of a somatotropin binding protein found in sera of animals containing this protein is presented and that such is distinct from the somatotropin receptor. This distinction is found in the difference of amino acids found on the carboxy terminus of the somatotropin binding protein. Thus, the isolation of the specific somatotropin binding protein is used as a tool to isolate and characterize each species' somatotropin binding protein, per the examples provided herein. Another object of the invention is to provide an isolated DNA sequence coding for serum somatotropin binding protein. Further, it is an object of the present invention to provide a method for expressing somatotropin binding proteins. Additionally, the use of the specific unique site of the binding protein to raise antibodies and study the affect on growth is provided herein. The antibodies, especially monoclonal in particular, of the present invention do not react with the somatotropin receptor. Further, the antibodies raised against the proteins of the invention alter growth characteristics of the target animal thereby providing the affects necessary to enhance growth. These and other objects of the invention will become apparent by the more detailed description of the invention provided hereinafter.

## Summary of the Invention

The present invention relates to novel somatotropin binding protein present in the sera of animals. The primary amino acid sequence of the somatotropin binding protein is illustrated in Figure 1 in which rat somatotropin binding protein is shown (The first amino acids, 1-17, code for a signal sequence. See also Example 9). However, as would be expected, the expression and identification of other sera somatotropin binding proteins, specifically, such as porcine, supports the existence of discrete animal sera somatotropin binding proteins. Thus, the present invention relates not only to the specific protein identified in Figure 1, with and without the leader signal sequence, but to proteins having substantially the same amino acid sequence and substantially the same growth regulating activity as the protein illustrated in Figure 1. Further, the invention relates to the purified isolated DNA sequence encoding for serum somatotropin binding protein identified in Figure 5 (with and without the signal sequence) and to DNAs encoding substantially the same amino acid sequence and with substantially the same growth regulating activity as the protein in Figure 1. It is appreciated by those of ordinary skill in the art that other such proteins from other species, as well as other alternatives to the protein illustrated in Figure 1, can be expressed by the process of the present invention. Various expression systems may be used to produce varieties to these proteins but such varieties still result in a serum somatotropin binding protein with the biological growth regulating activity of the present invention. The resultant DNA sequences and resulting proteins having such substantially the same growth regulating activity as tested by the assays disclosed herein in the examples, are included within the scope of the invention. Those assays include monoclonal antibody studies, ligand binding studies and growth determining assays. Monoclonal antibody studies characterize and identify amino acid sequence and structures of those amino acid sequences in a protein. Ligand binding studies functionally identify a protein's particular biological activities. Growth assays again define biological activity in vivo. For instance, the monoclonal antibodies raised against the somatotropin binding protein of the invention enhance growth. As such, regulating the serum somatotropin binding protein, either with antibodies or antagonists may be a mechanism for enhancing growth characteristics of an animal.

The serum somatotropin binding proteins of the present invention are produced through an expression vector comprising a DNA sequence encoding for serum somatotropin binding protein, a biologically active fragment of serum somatotropin binding protein or a biologically active mutant of serum somatotropin binding protein wherein one or more amino acids have been inserted, substituted or deleted in or from the amino acid sequence of serum somatotropin binding protein or its fragments. This includes DNA encoding for bovine, porcine, ovine, caprine, avian, fish or human serum somatotropin binding protein.

Additionally, the present invention includes a novel method for identifying serum somatotropin binding proteins of all animal species wherein a DNA probe selected from the DNA identified in Figure 5 or substantially the same DNA sequence as that identified in Figure 5 is used to isolate the appropriate DNA from other species, per the examples provided herein. The critical portion is the unique 17 amino acid tail, not found in other such DNA.

The proteins of the invention are formulated into pharmaceutically acceptable compositions, either alone or in combination with other growth regulating compounds such as somatotropins.

For purposes of the present invention all of the plasmids, DNA sequences, hybridoma cell lines and microorganisms deposited in connection with the present invention, except where specified to the contrary, are deposited in American Cyanamid Company's culture collection maintained in Princeton, New Jersey and are deposited with American Type Culture Collection in Rockville, Maryland 20952 USA, per the following:

| ATCC NUMBERS | | |
|---|---|---|
| Plasmid | Number | Date Registered |
| pRat6 | 67844 | November 4, 1988 |
| pRat12 | 67845 | November 4, 1988 |
| pRat7-12 | 67846 | November 4, 1988 |
| pRat5 | 67847 | November 4, 1988 |
| pRat1 | 67848 | November 4, 1988 |
| pSVL 7-6 | 67849 | November 4, 1988 |
| pSVL 7-12 | 67850 | November 4, 1988 |
| pRat7 | 67851 | November 4, 1988 |
| GHBP-4.3 | 10310 | December 15, 1989 |
| pET 7-6m | 68205 | January 19, 1990 |

All the above-listed deposits are accepted under the provisions of the Budapest Treaty and are available to public access upon the legally entitled time periods to do so. Further, replacements of the deposited samples will be made in the event the depository cannot dispense such requested sample.

Although the use of genetic engineering techniques lend themselves to the effective methods to produce the serum somatotropin binding proteins of the present invention it is equally to be noted that the present proteins encompass other methods of production, such as chemical synthesis.

Also for the purposes of the present invention and method of use, animal species include not only those specified such as porcine, but specifically include other animal protein species.

As is appreciated by those of ordinary skill in the art, the protein without the signal sequence is not available and only through the present invention has it been isolated, cloned and expressed for study.

## Brief Description of the Figures:

**Figure 1:** Amino acid sequence of rat somatotropin binding protein.

**Figure 2:** Restriction map of the cloned cDNAs for the rat somatotropin receptor and serum somatotropin binding protein. The coding region is shown as the larger of the open boxes. The smaller open box represents the 5′ and 3′ non-coding regions. H = HindIII, S = SmaI, B = BamHI, K = KpnI, Bg = BglII, Ss = SspI.

**Figure 3:** Sequencing strategy for pRat1. The arrows indicate the sequence data obtained from one reaction. H3 = HindIII, S = SmaI, B = BamHI, Sc = ScaI.

**Figure 4:** Sequencing strategy for pRat7-12. Symbols as described in Figure 3. K = KpnI, B = BglII, Ss = SspI.

**Figure 5:** Nucleotide sequence and amino acid sequence for the serum somatotropin binding protein. The nucleotide sequence is numbered beginning with the A of the first methionine (ATG codon) of the long open reading frame. Two sequences are shown upstream of the ATG for initiation of translation and are representative of the sequences of pRat1 and pRat6 cDNA clones in this region.

**Figure 6:** Nucleotide sequence and deduced amino acid sequence for the rat somatotropin receptor. The nucleotide sequence is numbered beginning with the A of the first methionine (ATG codon) of the long open reading frame.

**Figure 7:** Comparison of the nucleotide sequence and amino acid sequence of the somatotropin receptor and the serum somatotropin binding protein surrounding the region of divergence. The arrow indicates the point of divergence. R indicates the sequence of the somatotropin receptor and BP indicates the sequence of the serum somatotropin binding protein. The darker shading represents the putative transmembrane domain. The lighter shading represents the 17 amino acid residues present at the carboxy terminus of the somatotropin binding protein which are not present in the somatotropin receptor.

**Figure 8:** Schematic of the rat somatotropin receptor and the rat somatotropin binding protein. The hydropathy plot is from the method of Kyte and Doolittle with a window of 10 residues. The signal sequence (amino acid 1 throught 17 also is shown).

**Figure 9:** Northern type analysis. Each lane contains 3 μg of liver poly A(+) mRNA from female Sprague Dawley rats. The positions of 28S and 18SrRNA are indicated on the left. The sizes of the hybridizing species are shown on the right. Lane a is hybridized with probe A. Lane b is hybridized with probe B. Lane C is hybridized with probe C.

**Figure 10:** S1 nuclease analysis. Lanes a and b contain 3′ end labelled somatotropin receptor probe (GHR). Lanes c and d contain 3′ end labelled somatotropin binding protein probe (BP). Lanes a and c have no added RNA. Lanes b and d each contain 3 µg of liver poly A(+) mRNA from female Sprague Dawley rats.

**Figure 11:** Schematic of construction of pRat7-6. B = BamHI, E = EcoRI.

**Figure 12:** Schematic of construction of pSVL7-6. B = BamHI, E = EcoRI, S = SmaI.

**Figure 13:** Schematic of construction of pRat7-12. B = BamHI, E = EcoRI.

**Figure 14:** Schematic of construction of pSVL7-12. B = BamHI, E = Ecorl, S = SmaI.

**Figure 15:** Competition binding curve of the expressed rat somatotropin receptor cDNA in cos-7 cells. A constant amount of $I^{125}$ labelled bovine somatotropin is incubated with increasing amounts of unlabeled bovine somatotropin. Insert, Scatchard plot of the same data.

**Figure 16:** Saturation binding curve of the expressed rat somatotropin binding protein in cos-7 cells. Insert, Scatchard plot of the same data.

**Figure 17:** Northern type analysis. Each lane contains 3 µg of liver poly A(+) mRNA from various species of animals. Probe BP is the cDNA pRAT7-6. Probe R is from nucleotides 936 to 1674 of pRat7-12.

**Figure 18:** The deduced amino acid sequence of various species of somatotropin (also referred to as GH) receptor is provided.

**Figure 19:** Northern analysis of a variety of mRNAs from rat tissue is performed and indicate differences in levels dependent on the tissue studies. 3.0 µg of poly A(+) RNA are electrophoresed per lane. Liver tissue is from fetal rats at 18 days of development, 1 day old newborns, and 1 to 10 week old animals. Male and female livers are pooled to make RNA from fetal through 3 weeks of age, and all other tissues are from female Sprague Dawley rats. Tissues other than liver are from 8 week old animals.

**Figure 20:** The specific interaction of GHBP-4.3 monoclonal antibody with rat somatotropin binding protein is shown. Cell extracts from E. coli bacteria with the expression plasmid which directs the synthesis of rat somatotropin binding protein are electrophoresed on 15% polyacrylamide gel, transferred onto filter paper and probed with GHBP-4.3 monoclonal antibody and later with $^{125}I$ protein A and autoradiographed. Lane 2 contains proteins from E. coli cells which express rat somatotropin binding protein. Lane 1 contains E. coli extract with the expression plasmid with rat somatotropin binding protein sequences in reverse orientation. As shown, bacteria with rat somatotropin binding protein sequences in correct orientation can express a protein recognized by GHBP-4.3 monoclonal antibody which migrates at 30 Kd. Molecular weight markers are shown on the right (43Kd - ovalbumin; 29Kd - carbonic anhydrase; 18 Kd - beta-lactoglobulin).

**Figure 21:** The figure depicts the specific interaction of GHBP-4.3 monoclonal antibody with somatotropin binding protein in blood. One µ1 of plasma from each animal is electrophoresed on 15% polyacrylamide gel and blotted on Immobilon-P filter paper. The blot is probed with GHBP-4.3 monoclonal antibody and later alkaline phosphatase-labelled anti-mouse secondary antibody. The Western Blot then is developed for alkaline phosphatase activity. As shown, GHBP-4.3 monoclonal antibody recognizes a somatotropin binding protein in the rat (Lane 1), pig (Lane 4) and mouse (Lane 6) plasma. The presence of mouse immunoglobulin in plasma causes an extra band of 55Kd to interact with immunoglobulin secondary antibody. Molecular weight markers are shown on the right (84 Kd -fructose-6-phosphatese; 58 Kd - pyruvate kinase; 48 Kd -fumarase).

**Figure 22:** The specific binding of GHBP.-4.3 monoclonal antibody to a somatotropin binding protein/somatotropin complex is illustrated. The complex of rat somatotropin binding protein and $^{125}I$-somatotropin is immunoprecipitated with GHBP-4.3 monoclonal antibody and formalin-fixed Staph A. The complex is dissociated in sample buffer and electrophoresed on 15% polyacrylamide gel. The gel then is fixed, dried and autoradiographed. The autoradiogram shows the radioactive somatotropin which precipitated. The Figure shows the immunoprecipitation of the somatotropin binding protein/somatotropin complex with SP2/0 antibody (Lane 1), SP2/0 antibody and excess unlabelled somatotropin (Lane 2), GHBP-4.3 monoclonal antibody (Lane 3) and GHBP-4.3 monoclonal antibody and excess unlabelled somatotropin (Lane 4). In this Figure, somatotropin is indicated by "GH".

**Figure 23:** The effect of GHBP-4.3 monoclonal antibody on the growth of Balb/C mice in two separate experiments is shown:

23A: Three week old Balb/C mice are injected with on µg of GHBP-4.3 monoclonal antibody (Δ - Δ) or kept as untreated control (● - ●) and their weight gain monitored for two weeks. Each point represents the average weight gain of 10 mice.

23B: Three week old mice are treated with 100 µg GHBP-4.3 monoclonal antibody + 10 µg somatotropin (● - ●) or 10 µg control mouse IgG (Δ - Δ) or 10 µg somatotropin alone (O - O) and their

weight gain monitored for 35 days. Each point represents the average weight gain of four mice.

## Detailed Description of the Invention

The present invention relates to serum somatotropin binding protein. The species analyzed in greatest detail in this disclosure is the rat, although it is recognized that a similar protein exists in other species. Therefore, the invention includes bovine, ovine, porcine, caprine, avian and human such proteins, among others. Specifically, porcine somatotropin binding protein most relates to the unique protein described herein with greatest detail. Evidence in the scientific literature suggests that a serum somatotropin binding protein in humans and rabbits is actually derived from the somatotropin receptor by proteolytic cleavage and that the novel protein of the present invention does not exist as a distinct molecular entity. The present invention, on the other hand, discloses the structure of cDNA clones isolated from a rat liver cDNA library which encodes a soluble, secreted, somatotropin binding protein homologous to the extracellular domain of the somatotropin receptor in direct contrast to the published expectations. Further, the expression of the serum somatotropin binding protein in heterologous systems is provided in the following examples which illustrate the invention but are not limitative thereof.

## Example 1

cDNAs are isolated by screening of liver cDNA libraries. Two oligonucleotides, designated GHR1 and GHR2, are synthesized based on the published sequence of the rabbit liver somatotropin receptor (Leung et. al., 1987). The sequence of GHR1 is 5′ GAGCCCAAATTCACCAAGTGCCGTTCACCTGAACTAGAGAC 3′ and corresponds to positions 148 through 188 of the sequence of the published rabbit liver somatotropin receptor (Leung et. al., 1987). The sequence of GHR2 is 5′ GCACCTTCTCAGCTCAGCAATCCAAATTCACTGGCAAAC 3′ and corresponds to positions 1411 through 1449. Two libraries are used, a rabbit liver cDNA library and a rat liver cDNA library. The rabbit library is prepared by the method of Gubler and Hoffman (1983) using rabbit liver poly A(+) mRNA except that random hexanucleotides are used as a primer for first strand synthesis with AMV reverse transcriptase. Second strand synthesis is with RNase H, E. Coli DNA polymerase and E. Coli DNA ligase. The ECoRI linker double stranded cDNA is size selected (only fragments greater than 500 base pairs are taken for further analysis) and then cloned into bacteriophage λgt11. 2.7 x $10^6$ independent transformants are obtained, of which 63% are recombinant. The rat liver library is purchased from Clontech (Palo Alto, CA.). The first strand synthesis is oligo dT primed and contains 8.4 x $10^5$ independent clones of which 81% are recombinant. Both libraries are plated at a density of 150,000 clones/150mm plate and 6 plates of each are prepared. Duplicate lifts are taken from each plate. The oligonucleotides, GHR1 and GHR2, are end labeled with $\delta$-$^{32}$P-ATP using poly-nucleotide kinase. The filters are prehybridized in Church buffer (Church buffer is 7% (w/v) sodium dodecyl sulfate, 0.5% (w/v) bovine serum albumin, 0.5M $Na_2$ $HPO_4$ pH7.2, 1mm EDTA) at 65°C with shaking, for 30-60 minutes. Hybridization, at a concentration of 1-2 ng/ml labeled oligonucleotide, is in Church buffer at 65°C overnight, with shaking. The filters are washed twice, 20 minutes each, in 2 x SSC, 0.1% (w/v) SDS at room temperature and twice, 20 minutes each, in 1 x SSC, 0.1% (w/v) SDS at 55°C 1 x SSC is 0.15M NaCl, 0.015M $Na_3$ citrate pH7.0. The filters are exposed to x-ray film with lighting plus intensifying screens at -70°C. Table I illustrates the results. Six positives are plaque purified from the rat liver library using GHR1 as the probe. A second round of screening is performed on the rat liver cDNA library to isolate clones which correspond to the intracellular region of the somatotropin receptor. A rabbit liver cDNA clone, pRA5, which corresponds to nucleotides 1031 to 1531 of the published rabbit liver somatotropin receptor sequence is used as a probe. pRA5 is isolated from the rabbit liver cDNA library using the GHR2 oligonucleotide as probe. The insert is excised from the bacteriophage λgt11 vector by cleavage with ECoRI and the cDNA containing fragment is gel purified and cloned into pGEM3. Two rat liver cDNA clones, positive using pRA5 as probe, are plaque purified. All of the rat liver cDNAs are excised from the bacteriophage λgt11 vector by cleavage with ECoRI. The fragments are purified by agarose gel electrophoresis and cloned into the EcoRI site of pGEM3 (Promega Biotec, WI.).

TABLE I

| Library | GHR1 Posi | GHR2 Positives | GHR1 & GHR2 Positives |
|---|---|---|---|
| rat | 19 | 0 | 0 |
| rabbit | 18 | 82 | 1 |

## Example 2

A restriction map of the rat liver cDNAs cloned into pGEM3 is determined. The ends of each clone are sequenced using the Sanger dideoxy chain termination method (Sanger et. al., 1977) using the SP6 promoter primer and T7 promoter primer. Based on this analysis, four clones are picked for further investigation. These clones are designated pRat1, pRat6, pRat7 and pRat12. pRat1 and pRat6 are isolated using the GHR1 oligonucleotide as probe. pRat7 and pRat12 are isolated using pRA5 as probe. The clones are aligned as depicted in Figure 2 based on the restriction maps, preliminary sequence data and comparison with the published rabbit liver somatotropin receptor sequence. They are separated into two classes. Class I contains pRat1 and pRat6. Class II contains pRat7 and pRat12. Class I is homologous to the sequence of the rabbit liver somatotropin receptor at the presumptive 5′ end but diverges at the presumptive 3′ end. Class II is homologous to the sequence of the rabbit liver somatotropin receptor at both the presumptive 5′ end and the presumptive 3′ end. Two clones, pRat1 and pRat12, representative of each class are taken for further sequence analysis.

The sequencing strategy of pRat1 is depicted in Figure 3. The sequencing is performed by the dideoxy chain termination method. Appropriate restriction endonuclease fragments are subcloned into pGEM3 and sequenced using the SP6 promoter primer and T7 promoter primer. To obtain complete nucleotide sequence of pRat1 on both strands, oligonucleotides corresponding to the relevant incomplete regions are synthesized and these are used as primers for the sequencing. The sequencing strategy for pRat12 is depicted in Figure 4. A complication arose during the preliminary sequence analysis of pRat12. pRat12 contains two non-related cDNAs which are ligated together during the cloning process and, as such, is a common cDNA cloning artifact. The extreme 5′ end of pRat12 corresponds to rat apolipoprotein cIII. This data is not presented in either Figures 2 or 4 and the beginning of pRat12, which is depicted in those figures, is determined by comparison to both the results of the 5′ end sequencing of pRat7 and the known sequence of rat apolipoprotein cIII. A relevant hybrid of pRat7 and pRat12 is used for the sequencing project. This clone is depicted as pRat7-12. Appropriate restriction fragments from pRat7-12 are subcloned into pGEM3. The sequencing is performed by the Sanger dideoxy chain termination method using the SP6 promoter primer and T7 promoter primer. To obtain complete nucleotide sequence of pRat7-12 on both strands, oligonucleotides corresponding to the relevant incomplete regions are synthesized and these are used as primers for the sequencing. The final sequences for both clones are assembled using Microgenie software (Beckman Instruments, Palo Alto, CA.).

The complete DNA sequence of pRat1 is shown in Figure 5. The 5′ end of pRat6 is divergent from pRat1. The region of non-homology is shown. The 3′ ends of pRat1 and pRat6 are identical. The complete nucleotide sequence of pRat7-12 is shown in Figure 6. Analysis of the sequence reveals several features:

1. pRat1 contains one large open reading frame of 279 amino acids.

2. pRat1 is divergent from pRat6 11 nucleotides upstream of the ATG (start codon) for the 279 amino acid open reading frame. The divergence is therefore contained within the 5′ non-coding region and probably represents alternative transcription start sites.

3. pRat7-12 contains one open reading frame of 638 amino acids.

4. pRat1 and pRat7-12 are identical in sequence until nucleotide 787. (Numbering is from the A of the ATG sequence at the beginning of the open reading frame.) The sequence diverges after nucleotide 787 and there is no significant homology between pRat1 and pRat7-12 after that point of divergence (Figure 7).

5. pRat7-12 encodes a protein consistent with the properties of the somatotropin receptor. Analysis of the protein hydrophobicity profile (Figure 8) demonstrates that it likely contains a signal sequence located between amino acids 1 through 17 at the amino terminus and a single transmembrane domain located between amino acids 264 to 289. Homology comparisons between this putative rat somatotropin receptor and the published sequence of the rabbit liver somatotropin receptor indicates 70% identify.

6. pRat1 encodes a protein consistent with the properties of a soluble secreted serum somatotropin binding protein. Analysis of the protein hydrophobicity profile (Figure 8) demonstrates that it likely contains a signal sequence, located through amino acids 1 through 17. pRat1 diverges from pRat7-12 before the predicted transmembrane domain. The point of divergence is after amino acid 262. In place of the hydrophobic transmembrane domain present in pRat7-12 is a 17 amino acid hydrophilic region. This sequence is not present anywhere on the putative rat somatotropin receptor.

## Example 3

The structure of the cDNAs are representative of the structure of the mRNA present in rat liver. Two techniques are used to establish the structure of mRNA isolated from rat liver homologous to the isolated cDNAs. Figure 9 represents Northern type analysis of rat liver mRNA using three separate probes. Cytoplasmic RNA is prepared from the liver of female Sprague Dawley rats and poly A(+) RNA selected by chromatography on oligo dT cellulose. 3 $\mu$g of poly A(+) RNA per lane is electrophoresized on a 1% agarose gel containing formaldehyde. The RNA is transferred to a nylon filter. Each lane is hybridized separately to one of three probes. Probe A corresponds to nucleotides 91 to 382 of pRat7-12 (numbering is as described previously). This probe is present in both Class I and Class II cDNA clones. Probe B corresponds to nucleotides 936 to 1674 of pRat7-12. This probe is present only in Class II cDNA clones. Probe C corresponds to nucleotides 787 to 944 of pRat1. This probe is present only in Class I cDNA clones. The probes are labeled using the random priming technique (Feinberg and Vogalstein, 1983) with $\alpha^{-32}$P-dCTP as the labeled nucleotide. The filters are prehybridized and hybridized in Church buffer at 65°C. Hybridization is overnight. The filters are washed twice, 10 minutes each, in 2 x SSC, 0.2% SDS at room temperature, twice, 10 minutes each, in 0.2 x SSC, 0.2% SDS at room temperature and twice, 10 minutes each, in 0.2 x SSC, 0.2% SDS at 60°C. The filters are exposed to x-ray film with lighting plus intensifying screens at -70° C. Figure 9 illustrates the results. Probe A recognizes two mRNAs, of 4.75 kb and 1.2 kb. Probe B only recognizes the 4.75 kb mRNA and Probe C only recognizes the 1.2 kb mRNA species. These results indicate that Class I cDNAs are encoded by a 1.2 kb mRNA and that Class II cDNAs are encoded by a 4.75 kb mRNA. The cDNAs are, therefore, representative of the structure of the mRNAs present in rat liver and, further, that Class I and Class II are separate molecular classes.

To determine to the nucleotide level the structure of Class I and Class II mRNAs and their point of divergence, S1 nuclease mapping is performed using 3′ end labeled probes. Two probes are constructed. The first probe is prepared from pRat1-5 and is designated BP. This plasmid, pRat1-5, is a HindIII subclone of pRat1 inserted at the HindIII site of pGEM3. The HindIII fragment from pRat1 extends from the HindIII site at position 382 through to position 944 at the 3′ end of the cDNA and then extends to the HindIII site at the end of the polylinker. The orientation of the pRAT1-5 clone is such that the HindIII site at position 382 is adjacent to the polylinker sequence in pGEM3. To prepare the BP probe, pRat1-5 is cut with BamH1 and then incubated with $\alpha^{32}$PdGTP (3000 Ci/mmol) in the presence of the Klenow fragment of E. Coli DNA polymerase I. After an appropriate time of incubation, the Klenow enzyme is inactivated by heating at 65°C. The mixture is then cleaved with KpnI and electrophoresized on a 1% agarose gel. The 455 nucleotide fragment is isolated. This fragment is 3′ end labeled at position 501 with G. The second probe is prepared from pRat12-19 and is designated GHR. This plasmid, pRat12-19, is a HindIII at position 382 to BglII at position 936 subclone of pRat12 inserted between the HindIII and BamH1 sites of PGEM3. To prepare the GHR probe, pRat12-19 is cut with BamH1 and EcoRI and then incubated with $\alpha^{32}$PdGTP (3000 Ci/mmol) in the presence of the Klenow fragment of E. Coli DNA polymerase I. After an appropriate time of incubation, the Klenow enzyme is inactivated by heating at 65°C. The mixture is electrophoresized on a 1% agarose gel. The 461 nucleotide fragment is isolated. This fragment is 3′ end labeled at position 501 with G. Each probe is hybridized separately with or without 3 $\mu$g rat liver poly A(+) mRNA. After denaturation for 10 mins at 80°C, hybridization is at 50°C overnight in 80% formamide, 0.4MNaCl, 20mMPIPES pH6.8. After hybridization, the mixture is treated with S1 nuclease. S1 nuclease specifically degrades single stranded nucleic acids. The mixture is then electrophoresized, after denaturation, on a 4% acrylamide (20:1 acylamide:bis acylamide ratio)-50% urea gel. After electrophoresis the gel is subjected to autoradiography at -70°C with an intensifying screen. The result is shown in Figure 10. Probe GHR in the presence of no added mRNA has a fragment of 461 nucleotides which comigrates with the untreated GHR probe. This represents residual annealing of the probe to itself. In the presence of rat liver poly A(+) mRNA two additional fragments of 441 and 288 nucleotides are present. The 441 nucleotide band would correspond to mRNAs completely homologous to the probe over the entire length of the cDNA, consistent with a Class II mRNA. The 288 nucleotide band would correspond to a mRNA which diverges at position 788, consistent with a Class I mRNA. Probe BP in the presence of rat liver poly A(+) mRNA recognized two fragments of 444 and 288 nucleotides. The 444 nucleotide band corresponds to mRNA completely homologous to the probe over the entire length of the cDNA, consistent with a Class I mRNA. The 288 nucleotide band corresponds to a mRNA which diverged at position 788, consistent with a Class II mRNA. The relative ratios of Class I and II mRNAs revealed by the GHR and BP probes are consistent with each other and further are consistent with the Northern type analysis. Taken together, the mRNA analysis reveals the presence of two classes of poly A(+) mRNA present in rat liver consistent with the structure of the two classes of DNA represented by pRat1 and pRat7-12.

## Example 4

The cDNAs, therefore, when expressed in an appropriate system, are capable of binding somatotropin in a specific, saturable, high affinity manner. Further, pRat1 should encode a soluble binding activity and pRat7-12 should encode a membrane bound binding activity. Two expression constructs are made. pRat7-6 (depicted in Figure 11) contains the 5′ end of pRat7 (nucleotide positions -11 to 488). pRat7, pRat1 and pRat6 have identical sequence in this region. The 3′ end of pRat7-6 contains nucleotides 489 to 944 of pRat6. pRat6 and pRat1 have identical sequence in this region. Therefore, this construct should encode the soluble somatotropin binding protein. To show this pRat7-6 is cloned into the expression vector pSVL (Pharmacia) as depicted in Figure 12. The plasmid pSVL is designed for high level transient expression in eukaryotic cells. Genes inserted at the SmaI site are expressed from the first ATG codon using the SV40 late promoter. Transcripts are spliced and polyadenylated using the SV40 VP-1 processing signals. This expression construct is designated pSVL 7-6 (depicted in Figure 12). pRat7-12 contains the 5′ end of pRat7 (nucleotide position -11 to 488) and the 3′ end of pRat12 (nucleotide position 489 to 2536) and is depicted in Figure 13. This construct should encode the transmembrane somatotropin receptor. pRat7-12 is cloned into the expression vector pSVL as depicted in Figure 14. This expression construct is designated pSVL 7-12. Both expression constructs are transfected into Cos-7 cells using the calcium phosphate procedure. Cos-7 cells are monkey kidney CV-1 cells which contain and express the SV40 early region product, T antigen. T antigen is required for the replication of plasmids which contain the SV40 origin of replication and for expression of the SV40 late promoter. Both pSVL 7-6 and pSVL 7-12 are replicated and the products expressed in Cos-7 cells. Four days after transfection, the medium and washed cells from mock transfected Cos-7 cells, pSVL 7-6 transfected Cos-7 cells and pSVL 7-12 transfected Cos-7 cells are prepared. Using $I^{125}$ labeled bovine somatotropin, in the absence and presence of excess unlabeled bovine somatotropin, specific binding is assayed in the various fractions (Figures 15 and 16). No binding is obtained in either fraction with mock transfected Cos-7 cells. pSVL 7-6 transfected cells contain specific binding in the media and not associated with the cells. pSVL 7-12 transfected cells only contain specific binding associated with the cells and not in the media. Detailed kinetic analysis of the binding data reveal that the binding is of the expected high affinity and specificity. Thus, the identification of a novel serum somatotropin binding protein which is identical in sequence to the somatotropin receptor for the first 262 amino acids but then contains additional (17) amino acids - e.g. not present in the somatotropin receptor, is shown. This novel serum somatotropin binding protein is secreted from cells.

## Example 5

pRat 7-6 is digested with the restriction enzyme EcoRI and a 0.95 kb fragment containing the serum somatotropin binding protein is isolated. This fragment is ligated into the vector pGEM 3Z(f)+ (Promega Biotec, WI) which is digested with EcoRI. The orientation of the fragment is such that the 5′ end of the somatotropin binding protein gene adjoins the T7 RNA polymerase promoter. The resulting construct, pRat 3Z+ 7-6, is used for site-directed mutagenesis.

The strain E. Coli DH5α/pRat 3Z+ 7-6 is superinfected with the helper phage R408, and single stranded DNA is isolated. This DNA represents the negative strand with respect to the serum somatotropin binding protein coding region. An oligonucleotide of the sequence 5′GTCTCCAGCCATATGTTTCCT3′ is synthesized and annealed to the single stranded DNA. The second strand is completed using the Klenow fragment of E. Coli DNA polymerase and T4 DNA ligase. E. Coli strain DH5α is transformed, and the mixture is plated on LB-AMP plates. The colonies are transferred to nitrocellulose. Colony hybridization is performed in 1X Denhardts, 5X SSC and 150 μg/ml tRNA at 37°C using as probe the oligonucleotide which is end labeled with $^{32}P$ ATP. The filters are washed at 56°C in 3 M Tetramethyl ammonium chloride, 50 mM Tris pH 8, 2 mM EDTA and 0.1% SDS. Under these conditions, only colonies containing plasmids with the mutated sequence are labelled. Positive colonies are picked, from which plasmid DNA is made which may contain both mutant and wild type molecules. The transformation, hybridization and washing procedures are repeated, and a positive colony is again picked and used to make the purified mutant plasmid DNA. In this plasmid, called pRat 7-6m, the nucleotides at positions 49 and 51, a G and a C, respectively, of the wild type somatotropin binding protein gene are changed to a C and a T, respectively. This changes the resulting encoded polypeptide sequence from Asp to His at position 17 of the somatotropin binding protein, and creates a restriction endonuclease site for NdeI. This site falls immediately before a Met residue at position 18 (the NdeI site, CATATG, encodes Gln-Met). pRat 7-6m is digested with the restriction endonuclease NdeI, and the resulting 0.9 kb fragment is isolated. The NdeI cleavage sites are filled in using the Klenow fragment of E. Coli DNA polymerase, and EcoRI linkers of the sequence GGAATTCC are ligated to both ends of the fragment. The fragment is digested with EcoRI and repurified and is inserted into the EcoRI site of pATH1 (modified from K.R. Spindler, D.S.E. Rosser, and A.J. Berk (1984) J. Virol. 49:132-141). pATH1 is a vector that directs transcription in E. Coli of the trpE protein which can be fused to an exogenous coding region by insertion in-frame at the unique EcoRI site of pATH1. The resultant trpE fusion is driven from the inducible trpE promoter. The trpE/somatotropin binding protein fusion encodes amino acids 1-329 of trpE followed by Pro (CCT) encoded by the EcoRI linker and the newly created NdeI site, after which follows the complete coding sequence of the secreted serum somatotropin binding protein beginning with Met at position 18. The trpE/somatotropin binding protein fusion vector is called pATH7-6m.

The strain DH5α/pATH7-6m is cultured in M9 medium containing 0.4% casamino acids, 1% glucose and 100μg/ml amp at 37°C in shaker flasks. When growth reaches an $OD_{595}$ of 0.450, 3-beta-indoleacrylic acid (IAA) is added to 20 μg/ml. This induces transcription from the trpE promoter. After four hours, the cells are harvested and resuspended in one fifth volume 50 mM Tris pH 7.5, 5mM EDTA, 3 mg/ml lysozyme and 1 mM phenylmethylsulfonyl fluoride. After 2 hours on ice, NaCl is added to 0.3 M, followed after mixing by addition of NP-40 to 0.1%. After 30 minutes on ice, the viscous lysate is sonicated for 10 seconds at 350 W and centrifuged for 10 mins at 12,000 xg. The pellet, consisting of insoluble inclusion bodies is washed once in 1 M NaCl and 10 mM Tris pH 7.5 and once in 10 mM Tris pH 7.5. This material is dissolved in 4 M urea, diafiltered and purified using a trpE antibody affinity column. The purified fusion protein is used to immunize rabbits and mice for the production of polyclonal and monoclonal antibodies to the serum somatotropin binding protein. Alternatively, the specific endoprotease Factor Xa cleaves the purified fusion protein after the unique Gly-Arg sequence, found at position 326 of the trpE protein. This cleavage results in the formation of a 37 kD trpE fragment and a 32 kD somatotropin binding protein fragment, which includes four additional amino acids Ala-Arg-Ile-Pro at its amino terminus. These fragments are separated on SDS-polyacrylamide gels containing urea, and the somatotropin binding protein fragment is electroeluted prior to use as an antigen. Alternatively, the serum somatotropin binding protein is purified in active form by standard biochemical techniques.

The EcoRI fragment from pATH7-6m, containing the serum somatotropin binding protein, is inserted into the bacterial expression vector pEX2 (Clontech, CA). This construct, pEX7-6m, is used to transform the E. Coli strain N4830-1, which is grown at 32°C in M9 medium containing 0.4% casamino acids, 1% glucose and 100 μg/ml ampicillin. Upon induction by increasing the temperature to 39°C, the lambda $P_R$ promoter drives the transcription of a beta-galactosidase fusion protein consisting of the amino terminal 1006 residues of the E. Coli lacZ protein fused to the entire secreted form of the serum somatotropin binding protein. This fusion protein is isolated by the same method as described above, except anti-beta-galactosidase immunoaffinity columns are used in purification.

The NdeI fragment from pRat7-6m encoding the serum somatotropin binding protein is inserted into the NdeI site of the bacterial expression vector pRO211 (described in EP 173280). The orientation of the fragment is such that transcription of the serum somatotropin binding protein gene is driven by the lambda $P_L$ promoter. This plasmid is called pRO7-6m. The lambda $P_L$ promoter in pRO7-6m drives transcription of the serum somatotropin binding protein gene upon temperature induction from 32-39°C This results in production of a protein identical in amino acid sequence to the wild type secreted somatotropin binding protein.

The E. Coli strain 4300/pRO7-6m is grown overnight in L-broth containing 100 μg/ml ampicillin. 5 ml of this culture is used to inoculate 100 ml of M9 medium containing glucose and ampicillin. After 24 hours of growth at 32°C, 20 ml is introduced into 9.5 liters of minimal medium containing glucose and ampicillin in a NBS fermentor. The fermentation proceeds at 32°C until an $OD_{595}$ of 30 is achieved, at which point the temperature is raised to 39°C. The induction continues for four hours until harvest.

The EcoRI fragment from pRat 7-6m is inserted into the EcoRI site of pKT280, in such orientation that the serum somatotropin binding protein gene is driven by the beta-lactamase promoter. This plasmid, called pKT7-6m, encodes a serum somatotropin binding protein fused to the beta-lactamase signal sequence. This polypeptide is secreted into the periplasmic space of E. Coli strain C600.

The E. Coli strain C600/pKT7-6m is grown in L-broth overnight and this culture is used to inoculate 10 liters of minimal medium containing ampicillin and glucose in a NBS fermentor. The fermentation continues until an $OD_{595}$ of 30 is achieved. The cells are harvested and periplasmic proteins are isolated.

## Example 6

Expression of the serum somatotropin binding protein in the yeast Sacccharomyces cerevisiae requires placing the binding protein coding DNA under the transcriptional control of a yeast promoter region. The GAL10 promoter, contained in plasmid pBM150 is selected, as transcription from this promoter is highly regulated (repressed by glucose) and inducible (by galactose).

pRat7-6 is digested with the restriction enzyme EcoRI and a 0.95 kb fragment containing the serum somatotropin binding protein DNA is isolated. Expression vector pBM150 is digested with the same enzyme and subsequently treated with calf intestinal phosphatase, to remove the phosphate moiety from the protruding 5′ ends of the linearized vector molecule. The binding protein-containing fragment is ligated into the linearized expression vector and plasmid clones containing vector with the binding protein DNA in the proper orientation are identified. The proper orientation is one in which the 5′ end of the serum somatotropin binding protein DNA is contiguous with the GAL10 promoter region. This plasmid, named pGRAT7/6 is introduced into yeast strain DC38 by glusulase transformation. Transformants are identified by the acquisition of uracil prototrophy. The somatotropin binding protein is synthesized by the addition of galactose to the media.

## Example 7

The expression of serum somatotropin binding protein described hereinabove is used to produce serum somatotropin binding protein of other species.

Cytoplasmic poly A(+) mRNA is isolated from livers of adult dairy cow, adult steer and adult female rat which had been freshly frozen in liquid nitrogen and stored at -70°C. Three µg of poly A(+) mRNA per lane is electrophoresized on a 1% agarose gel containing formaldehyde and transferred to a nylon filter. Three lanes, representing rat, cow and steer liver RNA are each probed with one of two probes, as shown in Figure 17.

The probe denoted BP corresponds to the cDNA clone of pRat7-6. This probe is present in both Class I and Class II cDNA clones. The probe denoted R corresponds to nucleotides 936 to 1674 of pRat7-12. This probe is present only in Class II cDNA clones. The probes are labelled using the random probing technique with $\alpha$-$^{32}$P-dCTP as the labelled nucleotide. The filters are prehybridized for 30 minutes and hybridized for 24 hours at 55°C in Church buffer. The filters are washed four times, 20 minutes each in 2 x SSC, 0.1% SDS, twice at room temperature, and twice at 50°C. The filters are exposed to x-ray film with lighting plus intensifying screens at -70°C for 6 days. Figure 17 illustrates the results. These results are in agreement with the data obtained from the rat in that bovine serum somatotropin binding proteins are present. The same procedures as in the previous examples are used to isolate the cDNA, construct expression vectors and express serum ovine, porcine, caprine, avian and human somatotropin binding protein.

## Example 8

The bovine somatotropin binding protein cDNA is expressed in heterologous systems as described in Example 5 for the rat somatotropin binding protein. Briefly, the first amino acid after the signal sequence of the bovine somatotropin binding protein is converted to a methionine residue and the surrounding nucleotides are changed to generate the sequence CATATG which is an NdeI site. The ATG is now the methionine codon for initiation of translation of recombinant somatotropin binding protein. This construct is manipulated for expression similar to the rat somatotropin binding protein as described in Example 5.

## Example 9

pRat 7-6m, as prepared in Example 5, encodes a recombinant rat GH BP (hereinafter GH BP is synonymous with serum somatotropin binding protein) in which a Nde I restriction site has been engineered at the methionine residue at position 18 of the molecule. The 0.9 kb fragment excised by digestion with restriction endonuclease Nde I is ligated into the vector pET3b (Rosenberg et al, 1987) at the unique Nde I site. Thus, the methionine residue at position 18 serves as the initiation site for translation in this plasmid, which is called pET 7-6m. The resultant GH BP molecule lacks the putative signal sequence of the wild type unprocessed molecule as predicted by Leung et al (1987). This methionine residue at position 18 is not necessarily the first residue of the processed serum GH BP found in rats, (the initial residue of the processed wild type molecule has been predicted to be the threonine residue at position 25 in rats by Mathews et al (1989) and has been shown by amino-terminal sequencing to be the corresponding threonine 25 residue in mouse by Smith et at (1989)). The present molecule nevertheless represents a functionally equivalent species (as evidenced by the use of a cleavage site in human and rabbit after residue 17 (Leung et al, Ig87)) and allows for translation of the present rat GH BP in bacteria without the addition of the required initiator methionine residue at a position which does not exist in the wild type molecule.

Transcription of the recombinant rat GH BP is driven by a phage T7 gene promotor that signals constitutive transcription by phage T7 RNA polymerase, which is not normally found in E. coli. Therefore, an E. coli strain, JM 109 (DE3) (Promega, Inc.) that has a copy of the T7 RNA polymerase gene in a lambda lysogen, is used for expression of the recombinant GH binding protein. This T7 RNA polymerase gene is under the control of an inducible promoter (lacUV5). Thus expression of the recombinant GH BP is induced by the addition of 0.4 mM IPTG into the medium (Studier and Moffatt, 1986). An additional plasmid, pLysS which contains genes for chloramphenicol resistance and T7 lysozyme (Moffatt and Studier, 1987), may be included in the expression strain. Since the presence of T7 lysozyme inhibits the action of T7 RNA polymerase, this serves to prevent pre-induction expression of the recombinant GH BP.

The strain JM 109 (DE3)/ET 7-6m is grown overnight in M9 medium containing ampicillin (100ug/ml), 1% casamino acids and 20 g/l glucose. This is introduced into a fermentor, grown at 37 degrees Celcius to an OD of about 15 and induced by adding IPTG to 0.4 mM. After 2.5 hours, cells are harvested and frozen. Cells are resuspended in water, homogenized with a polytron and sonicated to disrupt genomic DNA. The recombinant product, in the form of inclusion bodies, is pelleted, washed, solubilized at high pH and purified by ultrafiltration with hollow fiber filters and chromatography on ion exchange hydrophobic interaction or affinity columns.

## Example 10

Purified somatotropin binding protein prepared by expression of the DNA encoding the somatotropin binding protein in any of the systems described in the previous examples is mixed with an appropriate amount of another growth regulating molecule, especially somatotropin, or formulated alone with a pharmacologically and pharmaceutically acceptable carrier. The mixture is administered to animals and results in manipulation of the growth regulating activity of somatotropin.

## Example 11

cDNA clones of the GH receptor as per Example 1 (including the region of the GH receptor which represents the GH BP in pig, sheep, cow and chicken) are isolated from oligo dT primed cDNA libraries as described in Example 1. In this case, however, the probes consist of the present rat GH receptor cDNA clones isolated herein. Libraries are probed with fragments of pRat 7-12 (0.74 kb, from the Bgl II site at nucleotide 937 to the Hind III site at 1675 of the rat GH receptor coding sequence). Probes are labelled using the random priming technique as in Example 3. Filters are hybridized overnight at 55 degrees C and washed at 50 degrees C in 1 X SSC and 0.2% SDS. Clones are isolated as in Example 1 and analyzed as in Example 2. Figure 18 (sequences of rat, pig, sheep and cow compared) shows the deduced amino acid sequences of a region of the pig, sheep and cow GH receptor. They are compared with the corresponding amino acid sequence from the rat GH receptor: blank spaces represent identical amino acids to the rat sequence; amino acid substitutions are indicated; dashed represent a region not yet sequenced; asterisks denote deletions relative to the rat sequence.

## Example 12

The present experiment shows that the GH receptor and GH BP in rats are independently regulated at the level of RNA transcription. The binding protein in rat (and other species that have a specific binding protein mRNA) is a unique genetic entity, with physiological functions that are not purely dependent on production of the receptor. By inference, all species that have GH receptors and binding proteins should have this unique genetic entity since the function of the binding protein is probably similar in all species.

Northern analysis as described in Example 3 is performed on a variety of different mRNAs from rat tissue. Hybridizations are performed as in Example 3, using as probe the radioactively labelled 0.9 kb Eco RI fragment from pRat 7-6. Three micrograms of poly A(+) RNA is electrophoresed in each lane. Bands representing binding protein or receptor mRNA are indicated. Liver mRNA is shown through development from fetal through 10 weeks of age, and various tissues are shown from adult rat. The ratio of receptor to binding protein mRNA is dramatically different both through development in the rat and among various tissues in adult rats. For instance, equivalent levels of BP to R are seen in fetal liver, while a ratio of about 10:1 is seen in older animals. By contrast, higher levels of receptor than binding protein mRNA are observed in heart, kidney and muscle. Thus, independent regulation of GH binding protein and GH receptor mRNA exists in the rat.

## Example 13

Sequence analysis of cDNA clones representing the rat somatotropin receptor and the rat serum somatotropin binding protein reveal that the following nucleotide sequence is transcribed to make mRNA of somatotropin binding protein but not somatotropin receptor: GGA CCC AAG TTC AAT TCC CAG CAC CCA CAT CAA GAG ATT GAC AAC CAC CTG TAA. This DNA sequence translate as Gly-Pro-Lys-Phe-Asn-Ser-Gln-His-Pro-His-Gln-Glu-Ile-Asp-Asn-His-Leu, followed by a stop codon, indicating that it formed the carboxyl terminus of the somatotropin binding protein. Using a solid-phase peptide synthesis procedure (the Fmoc polyamide method), an 18-amino acid peptide was produced with the following sequence: Cys-Gly-Pro-Lys-Phe-Asn-Ser-Gln-His-Pro-His-Gln-Glu-IleAsp-Asn-His-Leu. This synthetic sequence is identical to the theoretical sequence mentioned herein except for an addition at position 1 of a Cys residue, which is included for the purpose of coupling the peptide to an antigenic carrier protein. The peptide preparation is analyzed for purity on a Vydac $C_{18}$ (4.6mm x 25cm) HPLC column, using a linear gradient over 30 minutes of 10-50% 0.1% TFA/$CH_3CN$ and 90-50% 0.1% TFA/$H_2O$ with a flow rate of 1.5 $cm^3$/minute. Detection is by UV at 230 nm. Amino acid analysis is performed by acid hydrolysis followed by thin layer chromatography to give the results shown in Table II:

TABLE II

| Amino Acid Analysis of Synthetic Peptide | | |
|---|---|---|
| | Number of Residues | |
| Amino Acid | Actual | Calculated |
| Asparagine & Aspartic acid | 2.90 | 3 |
| Cysteine | * | 1 |
| Glutamine & Glutamic acid | 3.10 | 3 |
| Glycine | 1.00 | 1 |
| Histidine | 2.77 | 3 |
| Isoleucine | 1.07 | 1 |
| Leucine | 1.06 | 1 |
| Lysine | 0.95 | 1 |
| Phenylalanine | 0.98 | 1 |
| Proline | 3.30* | 2 |
| Serine | 0.84 | 1 |
| Total | 17.97 | 18 |

* Cysteine degrades on acid hydrolysis to give a product which co-elutes with proline.

Fast atom bombardment mass spectrometry provides a molecular weight determination (positive ion spectrum gave $M + H^+$ at m/z 2100) and sequence confirmation.

## Example 14

The synthetic peptide is then conjugated with keyhole limpet hemocyanin (KLH) via the thiol group of the cysteine residue at position 1 of the peptide. Ten milligrams of the pure synthetic peptide is coupled to 8 mg of KLH using m-maleimidobenzoic acid N-hydroxysuccinimide ester as a heterobifunctional cross-linking agent to produce N-terminally bound peptide conjugates. The resultant conjugates are dialyzed, lyophilized, and stored at -20°C prior to use.

## Example 15

Balb/C mice, 6 to 10 weeks of age, are purchased from Charles River Breeding Laboratories, Wilmington, MA. These mice are immunized with 100µg KLH-peptide conjugate which is emulsified in complete Freund's adjuvant. These animals are boostered with 50µg of the same antigen every 3 weeks thereafter. Their spleens are removed 3 days after the last boosting and single cell suspensions of lymphocytes prepared. These lymphocytes are fused with NS-1 mouse myeloma cells [source?] lacking hypoxanthine phyosphoribosyl transferase (HPRT) with 50% polyethylene glycol, suspended in Dulbecco's modified Eagle medium (D-MEM) containing 20% fetal calf serum [?] (FCS), 0.175µg/ml aminopterin, 13.6µg/ml hypoxanthine, 3.88µg/ml thymidine and 50µg/ml gentamicin (HAT medium), and finally dispensed in 96-well culture plates. After being cultured for 10-14 days, supernatants of the several hundred hybridomas who survived due to the HPRT-positive phenotype of the lymphocytes are collected for antibody screening in a solid-phase ELISA. Antigens are dissolved in PBS and 1µg in 100µl are added to each well of a 96-well flat bottom polystyrene plate. After being incubated for one hour, the plate is washed 3 times with PBS containing 0.05% Tween-20 by an automatic plate washer (Dynatech Wash II, Chantilly, VA). Each well is dispensed with 200µl of 2% BSA (Sigma) and the plate is incubated for another hour. Test samples are added to the wells, incubated for 30 minutes, washed 6 times with PBS, and added with 100µl of alkaline phosphatase-conjugated goat anti-mouse IgG $F(ab')_2$ (Zymed Laboratories, South San Francisco, CA). The plate is again washed after a 30 minute incubation and 100µl of p-nitrophenyl phosphate (1mg/ml, Sigma) in 0.1M diethanolamine, pH 10.3, added as substrate for color development. Finally, the colormetric response is recorded as optical density (OD) by an ELISA plate reader at a wavelength of 405nm. Incubation procedure is always performed at 37°C.

Fourteen hybridomas designated GHBP-1 to GHBP-14 are determined to produce appropriate antibodies by solid phase ELISA and are further subcloned by a limited dilution procedure. A clone from hybridoma GH BP-4 is selected and designated GHBP-4.3 as exemplary for further study and is injected intraperitoneally into Balb/C mice that had been primed with pristane for the production of antibody-containing ascites. Samples of the hybridoma designated GHBP-4.3 have been deposited with the American Type Culture Collection and have been assigned accession number ATCC HB 10310.

Ascites are collected from the peritoneal cavities of mice and immunoglobulin (Ig) are purified by 50% ammonium sulfate precipitation technique. Alternatively, samples are diluted to 50% with binding buffer (3M NaCl, 1.5M glycine, pH 8.9) and loaded to a preparative Protein A Superose HR 16/5 column on a fast protein liquid chromatography (FPLC) system (Pharmacia, Inc., Piscataway, NJ). Non-Ig fraction is eluted from the column with the binding buffer and the bound Ig was subsequently collected by rinsing the column with 0.1M citric acid, pH 3. It is immediately neutralized to pH 7-8 with 2M Tris buffer, pH 8.2. Antibody prepared by both procedures are extensively dialyzed against PBS, concentrated by ultrafiltration (Amicon, Danvers, MA), aliquoted, and finally stored at -20°C until used.

## Example 16

The E. coli bacteria from Example 9 or the purified somatotropin binding protein from these bacteria are used for Western blot assay to determine the specific interaction of the monoclonals with somatotropin binding protein. The rat somatotropin binding protein expressed in these bacteria contains 262 amino acids and an apparent molecular weight of 30Kd as judged by SDS-PAGE. The E. coli lysate containing somatotropin binding protein is boiled in SDS-PAGE sample buffer containing 40mM Tris pH 7.4, 5mM EDTA, 3% SDS, 1ml 2-mercaptoethanol and electrophoresed on 15% acrylamide. The gel is then electroblotted on Immobilon-P filter paper (Millipore). The blot is probed with GHBP-4.3 monoclonal antibody raised against the 17 amino acids of the carboxy-terminal end of the binding protein. As shown in Figure 20, the monoclonal antibody specifically recognizes the somatotropin binding protein in the E. coli extract (Lane 2). The monoclonal antibody does not interact with any protein of E. coli which contains an expression plasmid in which the somatotropin binding protein sequence is in reverse orientation (such a strain is unable to express the somatotropin binding protein) (Lane 1).

## Example 17

GHBP-4.3 monoclonal antibody is used to identify the rat circulating somatotropin binding protein. For this, rat plasma is prepared by drawing 5ml of rat blood directly in 1ml 0.5M EDTA. 1µl of plasma is denatured in 20µl sample buffer (containing 50mM Tris pH 7.4, 5mM EDTA, 3% SDS, 1µl 2-mercaptoethanol) and heated for 3 minutes (100°C). The plasma sample is then electrophoresed on 15% discontinuous polyacrylamide gel and the proteins separated on the gel were electroblotted on Immobilon-P filter paper. The blot is incubated with GHBP-4.3 monoclonal antibody (50µl ascites in 10ml of 10mM Tris pH 7.4, 150mM NaCl, 5% nonfat milk and 0.05% sodium azide) for 2 hours at room temperature, washed in 10mM Tris, 150mM NaCl, and exposed to alkaline phosphatase labelled anti-mouse antibody for another 2 hours at room temperature. The blot is washed again and developed for alkaline phosphatase reaction with its substrate (NBT) (nitroblue tetrazolium chloride) + (BCIP) 5-bromo-r-chloro-3-indolyl phosphate p-toluidine salt in 100 mM $NaHCO_3$ + 1mm $MgCl_2$. As shown in Figure 21 (first Lane), the antibody specifically reacts with a protein with a molecular weight of 48 Kd. The size of this protein is in the expected range for somatotropin binding protein considering that the unglycosylated protein (which contains no post-translational modifications) has a calculated molecular weight of 30 Kd and that it contains carbohydrate chains as its post-translational modification. In contrast, the molecular weight of the somatotropin receptor is approximately 120Kd (leung et al 1987).

The cross-reactivity of GHBP-4.3 monoclonal antibody with the somatotropin binding protein of other animals has also been tested. For this experiment, the EDTA-treated plasma of various animals is subjected to SDS-PAGE and Western blotting. The blot is incubated with the monoclonal antibody and later with goat anti-mouse antibody labelled with alkaline phosphatase as described. The result of this experiment is shown in Figure 21 where a cross reacting species of the appropriate size is seen in pig plasma.

In order to show that the GHBP-4.3 monoclonal antibody does not bind to the somatotropin receptor, the cell lysate of clone 9 cells, a rat hepatoma cell line which has been shown to contain the somatotropin receptor (American Type Culture Collection, Rockville, Maryland) is also subjected to SDS-PAGE and Western blotting. The monoclonal antibody is found not to cross-react with the somatotropin receptor. This result is expected since the peptide antigen used to raise this MAB does not exist in the rat somatotropin receptor.

## Example 18

Fifty µl of EDTA-treated rat plasma is incubated overnight at 4°C with 1ml of 25mM Tris buffer containing 150mM NaCl, 5mM EDTA, 0.5% NP-40 (a detergent), 3% BSA, and 0.3µCi of $^{125}$I-bST (bovine somatotropin) (5ng) pH 7.5. GHBP-4.3 antibody is added (5µg) and the whole complex precipitated with formalin-fixed staph A bacteria for an additional hour at room temperature. The complex is washed 3 times in 0.5% NP-40, 25mM Tris pH 7.5, 150mM NaCl and 5mM EDTA and then dissociated by heating in 2X SDS-PAGE sample buffer for 3 minutes. The sample is electrophoresed on 15% SDS-PAGE, the gel was fixed, dried, and finally exposed to x-ray film for autoradiography.

As shown in Figure 22, after immunoprecipitation of the radioactive complex with GHBP-4.3 monoclonal antibody, a protein band of 20 Kd corresponding to the $^{125}$I-labelled somatotropin is observed, indicating that the antibody specifically recognizes somatotropin binding protein which binds to $^{125}$I-somatotropin (Lane 3). Antibodies derived from SP2/0 parental line and normal mice are used as controls and no protein bands or a very faint band is demonstrated (Lane 2). Addition of excess unlabelled somatotropin causes the disappearance of the radioactive somatotropin, indicating that the immunoprecipitation of somatotropin is dependent on the presence of somatotropin binding protein. Thus, GHBP-4.3 monoclonal antibody specifically binds to somatotropin binding protein after forming a complex with somatotropin. Addition of the monoclonal antibody at the same time as the radiolabelled somatotropin did not alter the pattern of immunoprecipitation, suggesting that this antibody does not interfere with the binding site of somatotropin to the binding protein.

## Example 19

To investigate the effect of the monoclonal antibody GHBP-4.3 in vivo, passive immunization is used. For this, the antibody is injected directly into young mice whose growth is followed by measuring weight gain. Figure 23A shows the results of such an experiment. Three groups of 10 mice/group are injected with:

1. 200 µl ascites fluid containing 1 mg of GHBP-4.3 monoclonal antibody per mouse.
2. 200 µl ascites fluid containing normal mouse polyclonal antibodies per mouse (as control).
3. Untreated control.

In this figure, it can be seen that the mice treated with GHBP-4.3 monoclonal antibody have a larger average weight gain than the mice which are not treated (After 14 days, the mice treated with GHBP-4.3 monoclonal antibody also had a larger average weight gain than the mice which are treated with normal mouse antibody).

In a similar experiment shown in Figure 23B, a combination of GHBP-4.3 monoclonal antibody and somatotropin is used to investigate the effect of the antibody on the growth rate. Groups of four mice each are treated with 100 µg GHBP-4.3 monoclonal antibody + 10 µg somatotropin, 10 µg somatotropin alone or 100 µg of control mouse IgG per mouse. As shown in Figure 23B, the mice receving GHBP-4.3 monoclonal antibody and somatotropin gain weight at a faster rate than control mice or the mice treated with somatotropin alone.

These two experiments indicate that the regulation of somatotropin binding protein has a profound influence on the rate of weight gain, and as such on growth characteristics.

## Example 20

The strain E. coli DH5alpha/pRat 32+ 7-6 contains the wild-type coding region for the rat GH BP, derived from a rat liver cDNA. The particular nucleotide sequence, due to degeneracy of the genetic code, can be changed somewhat in this plasmid by oligonucleotide directed mutagenesis without changing the polypeptide sequence of the mature protein. There are several reasons for which this may be done: to create convenient restriction enzyme sites in the plasmid for subsequent genetic manipulations; to improve the translation of the messenger RNA that is made from the DNA sequence (due to variations in the host translation machinery of different expression systems); to alter the activity of pre-processed forms of the protein, which would not affect the mature protein structure (such as changes in the signal sequence - a region which is proteolytically removed during secretion from the plasmid membrane). This is illustrated in Example 5 hereinabove.

Additionally, changes in the nucleotide sequence may be made which do change the polypeptide sequence of the mature protein. Example 5 illustrates this, where the mature protein produced in bacteria begins with a Met residue. Even though this Met residue at position 18 is contained in the wild type sequence, the mature BP in serum (shown by Smith et al in mice and predicted by Mathews et al in rats) begins with Thr(25). However, to produce a protein in bacteria, Met must be the initial amino acid. Thus, the actual serum version of the GHBP could not be made easily or in large amounts in E. coli. In any case, changes such as this and any number of other subtle changes may be made in the protein without changing its biological activity. Specific changes may nevertheless be made resulting in deletions, insertions or amino acid substitutions which do alter the biological activity of GH BP. Such alterations in biological activity may result in improved growth characteristics when introduced into animals as in Example 10.

## BIBLIOGRAPHY


Barnard, R. and Waters, M. J., Biochem. J., 237, 885-892 (1986).

Baumann, G., et al., J. Clin. Endo. Metab., 62, 134-141 (1986).

Baumann, G., et al., J. Clin. Endo. Metab., 65, 814-816 (1987).

Baumann, G. and Shaw, M. A., Biochem. Biophys. Res. Commun., 152, 573-578 (1988).

Baumbach, W. R., et al., Genes & Development, 3, 1195-1205 (1989).

Daughaday, W. H. and Trivedi, B., Proc. Natl. Acad. Sci., 84, 4636-4640 (1987).

Dunn, J. J. and Studier, F. W., J. Mol. Biol., 166, 477-535 (1983).

Eshet, R., et al., Israel J. Med. Sci., 20, 8-13 (1984).

Herington, A. C., et al., J. Clin. Invest., 77 1817-1823 (1986).

Hughes, J. P. and Friesen, H. G., Ann. Rev. Physiol., 47, 469-482 (1985).

Leung, D. W., et al., Nature, 330, 537-543 (1987).

Moffatt, B. A. and Studier, F. W., Cell, 49, 221-227 (1987).

Rosenberg, A. H., Lade, B. N., Chu, D., Lin S., Dunn, J. J. and Studier, F. W., Gene 56 125-135 (1987).

Smith, W. C., et al., Mol. Endo., 3, 984-990 (1989).

Spencer, S. A., et al., J. Biol. Chem., 263, 7862-7867 (1984).

Studier, F. W. and Moffatt, B. A., (1986). J. Mol. Biol. 189: 113-130.

Trivedi, B. and Daughaday, W. H., Endocrinology, 123, 2201-2206 (1988).

Ymer, S. I. and Herington, A. C., Mol. Cell. Endocrinol., 41, 153-161 (1985).

U.S. Patent 4,857,637.


**Claims**

1. A purified serum somatotropin binding protein containing the 1 through 17 amino acid carboxy terminal or equivalent carboxy terminal thereof.

2. The serum somatotropin binding protein according to Claim 1, wherein said protein is rat, bovine, porcine, ovine, caprine, avian, fish or human serum somatotropin binding protein.

3. Serum somatotropin binding protein having the amino acid sequence defined in Figure 1.

4. Serum somatotropin binding protein having the amino acid sequence defined in Figure 1 or substantially the same amino acid sequence and substantially the same growth regulating activity as the protein defined in Figure 1.

5. A purified isolated DNA sequence encoding for serum somatotropin binding protein.

6. A purified isolated DNA sequence encoding for serum somatotropin binding protein defined in Figure 5 or for a protein having substantially the same DNA sequence and substantially the same growth regulating activity as the protein defined of the DNA in Figure 5.

7. A purified isolated DNA sequence according to Claim 6 wherein said serum somatotropin binding protein is rat, mouse, bovine, porcine, ovine, caprine, avian, fish or human serum somatotropin binding protein.

8. A method for identifying serum somatotropin binding protein comprising: selecting a DNA probe as identified in Figure 5 or having a DNA sequence substantially the same as that defined in Figure 5 to isolate DNA from bovine, ovine, porcine, caprine, avian, fish or human species.

9. An expression vector comprising a DNA sequence encoding for serum somatotropin binding probe selected from the group of a biologically active fragment of serum somatotropin binding protein or a biologically active mutant of serum somatotropin binding protein wherein one or more amino acid residues have been inserted, substituted or deleted in or from the amino acid sequence of said serum somatotropin binding protein or its fragment.

10. An expression vector according to Claim 9 wherein said DNA encodes for rat, mouse, bovine, porcine, ovine, caprine, avian, fish or human serum somatotropin binding protein.

11. A pharmaceutical composition comprising an effective growth promoting amount of serum somatotropin binding protein and a pharmacologically and pharmaceutically acceptable carrier thereof.

12. A composition according to Claim 11, additionally comprising somatotropin.

13. A method for effecting growth in an animal comprising administering to said animal an effective amount of serum somatotropin binding protein.

14. A method according to Claim 13, wherein said animal is bovine, porcine, ovine, caprine, avian, fish or human.

FIG. I

```
                                     10                                                     20
Met Asp Leu Trp Arg Val Phe Leu Thr Leu Ala Leu Ala Val Ser Ser Asp Met Phe Pro
                                     30                                                     40
Gly Ser Gly Ala Thr Pro Ala Thr Leu Gly Lys Ala Ser Pro Val Leu Gln Arg Ile Asn
                                     50                                                     60
Pro Ser Leu Arg Glu Ser Ser Ser Gly Lys Pro Arg Phe Thr Lys Cys Arg Ser Pro Glu
                                     70                                                     80
Leu Glu Thr Phe Ser Cys Tyr Trp Thr Glu Gly Asp Asp His Asn Leu Lys Val Pro Gly
                                     90                                                    100
Ser Ile Gln Leu Tyr Tyr Ala Arg Arg Ile Ala His Glu Trp Thr Pro Glu Trp Lys Glu
                                    110                                                    120
Cys Pro Asp Tyr Val Ser Ala Gly Ala Asn Ser Cys Tyr Phe Asn Ser Ser Tyr Thr Ser
                                    130                                                    140
Ile Trp Ile Pro Tyr Cys Ile Lys Leu Thr Thr Asn Gly Asp Leu Leu Asp Glu Lys Cys
                                    150                                                    160
Phe Thr Val Asp Glu Ile Val Gln Pro Asp Pro Pro Ile Gly Leu Asn Trp Thr Leu Leu
                                    170                                                    180
Asn Ile Ser Leu Pro Gly Ile Arg Gly Asp Ile Gln Val Ser Trp Gln Pro Pro Pro Ser
                                    190                                                    200
Ala Asp Val Leu Lys Gly Trp Ile Ile Leu Glu Tyr Glu Ile Gln Tyr Lys Glu Val Asn
                                    210                                                    220
Glu Thr Lys Trp Lys Thr Met Ser Pro Ile Trp Ser Thr Ser Val Pro Leu Tyr Ser Leu
                                    230                                                    240
Arg Leu Asp Lys Glu His Glu Val Arg Val Arg Ser Arg Gln Arg Ser Phe Glu Lys Tyr
                                    250                                                    260
Ser Glu Phe Ser Glu Val Leu Arg Val Thr Phe Pro Gln Met Asp Thr Leu Ala Ala Cys
                                    270
Glu Glu Gly Pro Lys Phe Asn Ser Gln His Pro His Gln Glu Ile Asp Asn His Leu
```

RESTRICTION MAP OF RAT LIVER cDNAs

FIG.2

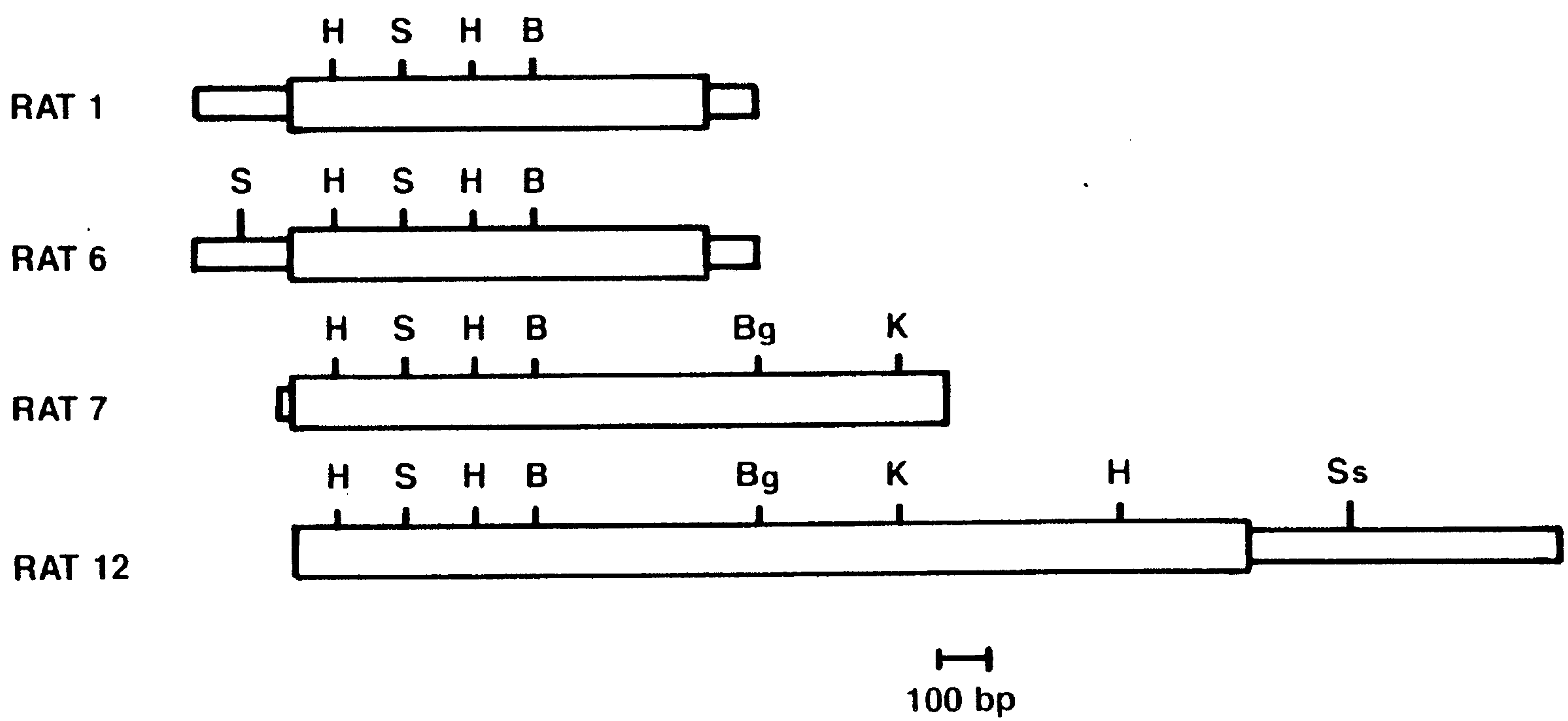

FIG.3

SEQUENCING STRATEGY FOR RAT 1

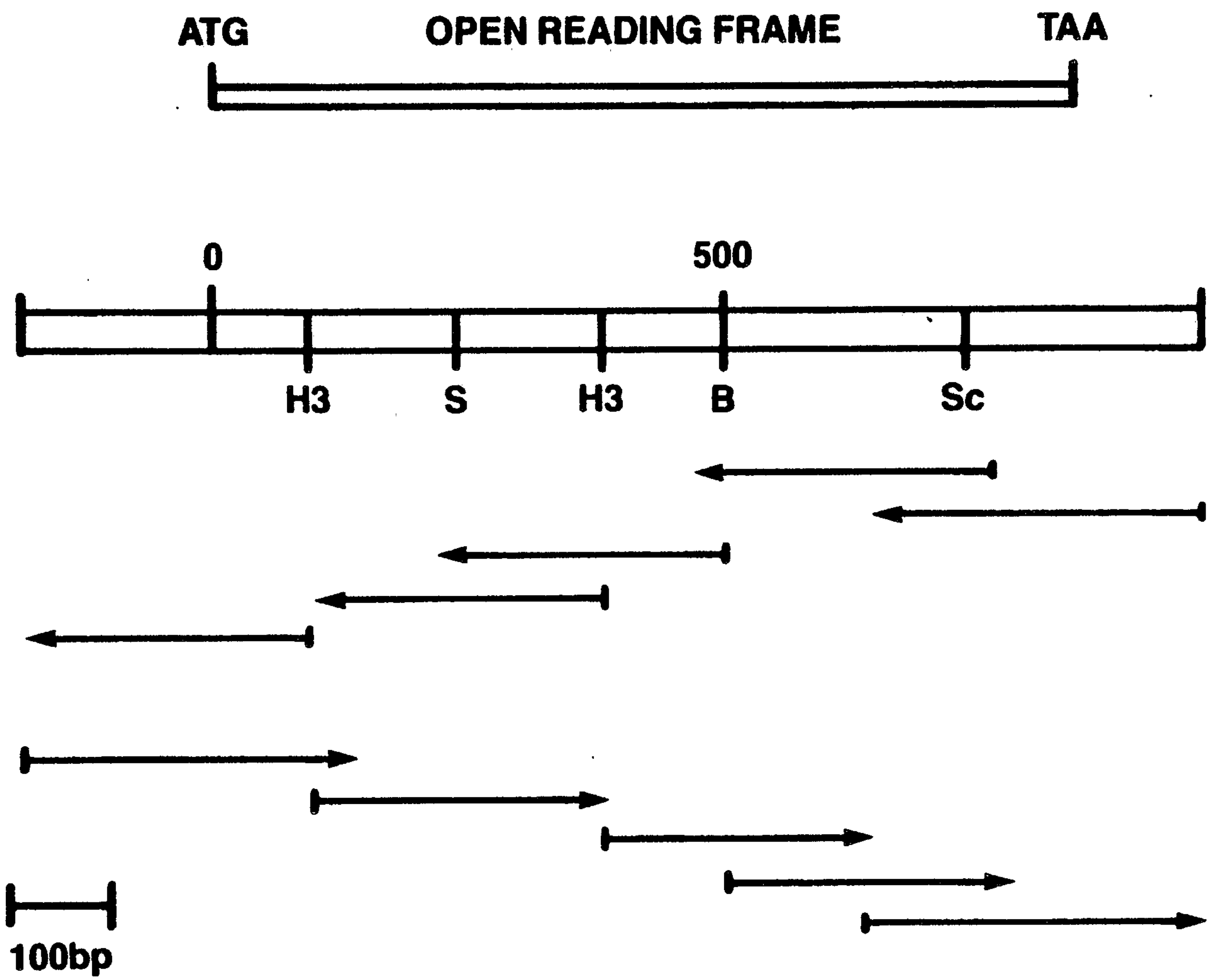

FIG.4

SEQUENCING STRATEGY FOR RAT 7-12

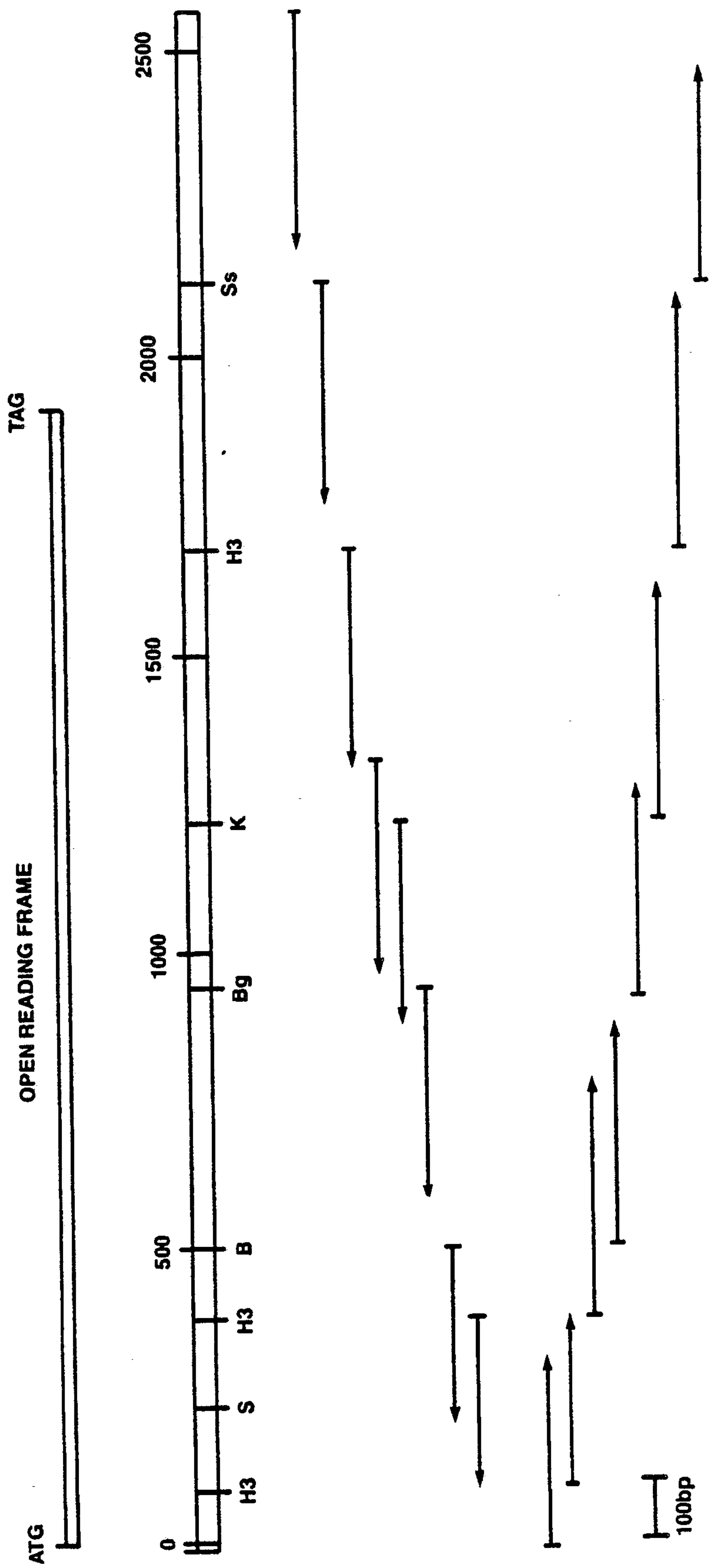

# FIG.5

RAT GROWTH HORMONE BINDING PROTEIN

```
              -191        -181        -171        -161        -151        -141        -131        -121
Rat 1                         CGTGGGAGAGACCACAAGATAGAGGATTATTAATTTATGGGGATTTTTTGGAGATTTATATACCCAA
                               :  :  : : :      : :   :  : :::       ::         : : :   :   :
Rat 6                 CGGCGGTCTAGGGAGCGGCGGCACTGGCAGAAGCGGCTGTTACAGCGGCGGTGGCGGCGACGGCTGTTGCTGAGCCC

   -111       -101       -91        -81        -71        -61        -51        -41        -31        -21        -11         -1
GCAACAACTCCTAGATGCTACAGACCAAGACACCAAGAAATAACCACGAAATTCTACTTTCAACCCTAGCTTCTCTACCAGATTTTAACTAAACAAGATCTTCTTGCAAAGGTCTCAGGT
    :  : ::  :  : : :   : :  :  :                                  ::: :::  :           :   :   : :   :     :  :::::::::::
CGGCAGCCGGCGAGGACCCCCGGGCTGGGGCCACGCGGCGCCTGAGGCTCGGCTCCAGCAGCCCCCAAGCGGACCGGAACCCGGCTCTGTCTCCCAAGGCGAACGTCCGAGGTCTCAGGT

        .         .        30         .         .        60         .         .        90         .         .       120
ATGGATCTTTGGCGGGTGTTCTTAACCCTGGCACTGGCAGTCTCCAGCGACATGTTTCCTGGAAGTGGGGCTACACCAGCTACTCTTGGCAAAGCTTCCCCGGTTCTGCAAAGAATTAAT
MetAspLeuTrpArgValPheLeuThrLeuAlaLeuAlaValSerSerAspMetPheProGlySerGlyAlaThrProAlaThrLeuGlyLysAlaSerProValLeuGlnArgIleAsn
        .         .       150         .         .       180         .         .       210         .         .       240
CCAAGCCTGAGGGAAAGTTCCTCTGGAAAGCCTCGATTCACCAAGTGTCGTTCCCCTGAACTGGAGACCTTTTCATGCTACTGGACAGAAGGGGATGATCATAATTTAAAGGTCCCGGGA
ProSerLeuArgGluSerSerSerGlyLysProArgPheThrLysCysArgSerProGluLeuGluThrPheSerCysTyrTrpThrGluGlyAspAspHisAsnLeuLysValProGly
        .         .       270         .         .       300         .         .       330         .         .       360
TCTATTCAGCTATACTATGCTAGAAGAATTGCTCACGAATGGACCCCGGAATGGAAAGAATGCCCTGATTATGTCTCTGCTGGAGCAAACAGCTGTTACTTCAACTCATCGTATACCTCC
SerIleGlnLeuTyrTyrAlaArgArgIleAlaHisGluTrpThrProGluTrpLysGluCysProAspTyrValSerAlaGlyAlaAsnSerCysTyrPheAsnSerSerTyrThrSer
        .         .       390         .         .       420         .         .       450         .         .       480
ATTTGGATACCCTACTGCATTAAGCTTACTACAAATGGTGATTTGTTGGACGAAAAGTGTTTCACTGTTGATGAAATAGTGCAACCTGATCCGCCCATTGGCCTCAACTGGACTTTACTA
IleTrpIleProTyrCysIleLysLeuThrThrAsnGlyAspLeuLeuAspGluLysCysPheThrValAspGluIleValGlnProAspProProIleGlyLeuAsnTrpThrLeuLeu
        .         .       510         .         .       540         .         .       570         .         .       600
AACATCAGTTTGCCTGGGATCCGTGGAGATATCCAAGTGAGTTGGCAGCCACCGCCCAGTGCCGATGTTCTGAAGGGATGGATAATTCTGGAGTATGAAATTCAGTACAAAGAAGTAAAT
AsnIleSerLeuProGlyIleArgGlyAspIleGlnValSerTrpGlnProProProSerAlaAspValLeuLysGlyTrpIleIleLeuGluTyrGluIleGlnTyrLysGluValAsn
        .         .       630         .         .       660         .         .       690         .         .       720
GAAACAAAATGGAAAACGATGAGCCCGATATGGTCAACATCAGTCCCACTGTACTCACTGAGACTGGATAAAGAGCACGAAGTGCGTGTGAGATCCAGACAACGGAGCTTCGAAAAGTAC
GluThrLysTrpLysThrMetSerProIleTrpSerThrSerValProLeuTyrSerLeuArgLeuAspLysGluHisGluValArgValArgSerArgGlnArgSerPheGluLysTyr
        .         .       750         .         .       780         .         .       810         .         .       840
AGCGAGTTCAGTGAAGTACTCCGTGTAACGTTTCCTCAGATGGACACACTGGCAGCATGTGAAGAAGGACCCAAGTTCAATTCCCAGCACCCACATCAAGAGATTGACAACCACCTGTAA
SerGluPheSerGluValLeuArgValThrPheProGlnMetAspThrLeuAlaAlaCysGluGluGlyProLysPheAsnSerGlnHisProHisGlnGluIleAspAsnHisLeuEND
        .         .       870         .         .       900         .         .       930         .
CACCAGCTCCAGAGGATCTGCCATCCCTAGCTTGCGGGCACCTGCATTCATATGCACATACATGCATACACATAATTAAAATTAATAAAATAAAATCGGAATTC
```

RAT GROWTH HORMONE RECEPTOR

# FIG.6

-11 -1
CGGTCTCAGGT
10 20 30 40 50 60 70 80 90 100 110 120
ATGGATCTTTGGCGGGTGTTCTTAACCCTGGCACTGGCAGTCTCCAGCGACATGTTTCCTGGAAGTGGGGCTACACCAGCTACTCTTGGCAAAGCTTCCCCGGTTCTGCAAAGAATTAAT
MetAspLeuTrpArgValPheLeuThrLeuAlaLeuAlaValSerSerAspMetPheProGlySerGlyAlaThrProAlaThrLeuGlyLysAlaSerProValLeuGlnArgIleAsn
130 140 150 160 170 180 190 200 210 220 230 240
CCAAGCCTGAGGGAAAGTTCCTCTGGAAAGCCTCGATTCACCAAGTGTCGTTCCCCTGAACTGGAGACCTTTTCATGCTACTGGACAGAAGGGGATGATCATAATTTAAAGGTCCCGGGA
ProSerLeuArgGluSerSerSerGlyLysProArgPheThrLysCysArgSerProGluLeuGluThrPheSerCysTyrTrpThrGluGlyAspAspHisAsnLeuLysValProGly
250 260 270 280 290 300 310 320 330 340 350 360
TCTATTCAGCTATACTATGCTAGAAGAATTGCTCATGAATGGACCCCGGAATGGAAAGAATGCCCTGATTATGTCTCTGCTGGAGCAAACAGCTGTTACTTCAACTCATCGTATACCTCC
SerIleGlnLeuTyrTyrAlaArgArgIleAlaHisGluTrpThrProGluTrpLysGluCysProAspTyrValSerAlaGlyAlaAsnSerCysTyrPheAsnSerSerTyrThrSer
370 380 390 400 410 420 430 440 450 460 470 480
ATTTGGATACCCTACTGCATTAAGCTTACTACAAATGGTGATTTGTTGGACGAAAAGTGTTTCACTGTTGATGAAATAGTGCAACCTGATCCGCCCATTGGCCTCAACTGGACTTTACTA
IleTrpIleProTyrCysIleLysLeuThrThrAsnGlyAspLeuLeuAspGluLysCysPheThrValAspGluIleValGlnProAspProProIleGlyLeuAsnTrpThrLeuLeu
490 500 510 520 530 540 550 560 570 580 590 600
AACATCAGTTTGCCTGGGATCCGTGGAGATATCCAAGTGAGTTGGCAGCCACCGCCCAGTGCCGATGTTCTGAAGGGATGGATAATTCTGGAGTATGAAATTCAGTACAAAGAAGTAAAT
AsnIleSerLeuProGlyIleArgGlyAspIleGlnValSerTrpGlnProProProSerAlaAspValLeuLysGlyTrpIleIleLeuGluTyrGluIleGlnTyrLysGluValAsn
610 620 630 640 650 660 670 680 690 700 710 720
GAAACAAAATGGAAAACGATGAGCCCGATATGGTCAACATCAGTCCCACTGTACTCACTGAGACTGGATAAAGAGCACGAAGTGCGTGTGAGATCCAGACAACGGAGCTTCGAAAAGTAC
GluThrLysTrpLysThrMetSerProIleTrpSerThrSerValProLeuTyrSerLeuArgLeuAspLysGluHisGluValArgValArgSerArgGlnArgSerPheGluLysTyr
730 740 750 760 770 780 790 800 810 820 830 840
AGCGAGTTCAGTGAAGTACTCCGTGTAACGTTTCCTCAGATGGACACACTGGCAGCATGTGAAGAAGATTTCCGGTTTCCATGGTTCTTAATTATTATCTTTGGAATATTTGGAGTAGCA
SerGluPheSerGluValLeuArgValThrPheProGlnMetAspThrLeuAlaAlaCysGluGluAspPheArgPheProTrpPheLeuIleIleIlePheGlyIlePheGlyValAla
850 860 870 880 890 900 910 920 930 940 950 960
GTGATGCTATTTGTAGTTATATTTTCAAAGCAGCAAAGGATTAAGATGCTGATTTTACCCCCAGTACCAGTTCCAAAGATTAAAGGGATTGATCCAGATCTTCTCAAGGAAGGAAAATTG
ValMetLeuPheValValIlePheSerLysGlnGlnArgIleLysMetLeuIleLeuProProValProValProLysIleLysGlyIleAspProAspLeuLeuLysGluGlyLysLeu
970 980 990 1000 1010 1020 1030 1040 1050 1060 1070 1080
GAGGAGGTGAACACCATCTTGGGCATTCATGATAACTACAAACCCGACTTCTACAATGATGACTCCTGGGTTGAGTTCATTGAGCTGGATATTGATGATGCGGATGAGAAGACTGAAGAG
GluGluValAsnThrIleLeuGlyIleHisAspAsnTyrLysProAspPheTyrAsnAspAspSerTrpValGluPheIleGluLeuAspIleAspAspAlaAspGluLysThrGluGlu
1090 1100 1110 1120 1130 1140 1150 1160 1170 1180 1190 1200
TCAGACACCGACAGACTTCTAAGCGATGACCAGGAGAAATCAGCTGGTATCCTTGGAGCAAAGGATGACGATTCTGGACGTACCAGCTGTTATGACCCTGACATTTTGGATACCGATTTC
SerAspThrAspArgLeuLeuSerAspAspGlnGluLysSerAlaGlyIleLeuGlyAlaLysAspAspAspSerGlyArgThrSerCysTyrAspProAspIleLeuAspThrAspPhe
1210 1220 1230 1240 1250 1260 1270 1280 1290 1300 1310 1320
CACACCAGTGACATGTGCGATGGTACCTCGGAGTTTGCTCAGCCGCAGAAGTTAAAAGCAGAAGCTGATCTCTTGTGCCTTGACCAGAAGAATCTGAAGAACTCGCCTTATGATGCTTCC
HisThrSerAspMetCysAspGlyThrSerGluPheAlaGlnProGlnLysLeuLysAlaGluAlaAspLeuLeuCysLeuAspGlnLysAsnLeuLysAsnSerProTyrAspAlaSer
1330 1340 1350 1360 1370 1380 1390 1400 1410 1420 1430 1440
CTTGGCTCTCTGCACCCCTCCATTACCCTGACAATGGAAGACAAACCACAGCCACTTCTGGGCAGTGAAACTGAGTCAACCCACCAACTCCCCTCTACACCAATGAGCAGTCCCGTGTCA
LeuGlySerLeuHisProSerIleThrLeuThrMetGluAspLysProGlnProLeuLeuGlySerGluThrGluSerThrHisGlnLeuProSerThrProMetSerSerProValSer
1450 1460 1470 1480 1490 1500 1510 1520 1530 1540 1550 1560
CTGGCAAACATTGACTTTTATGCCCAAGTAAGCGACATTACACCAGCAGGTGGTGTAGTCCTTTCTCCAGGCCAAAAGATTAAGGCAGGGTTAGCCCAGGGCAACACCCAGCTGGAGGTG
LeuAlaAsnIleAspPheTyrAlaGlnValSerAspIleThrProAlaGlyGlyValValLeuSerProGlyGlnLysIleLysAlaGlyLeuAlaGlnGlyAsnThrGlnLeuGluVal
1570 1580 1590 1600 1610 1620 1630 1640 1650 1660 1670 1680
GCCGCGCCCTGCCAAGAAAATTACAGCATGAACAGTGCCTACTTCTGTGAGTCAGATGCCAAAAAGTGCATCGCTGCGGCCCCTCACATGGAGGCCACGACATGTGTAAAACCAAGCTTT
AlaAlaProCysGlnGluAsnTyrSerMetAsnSerAlaTyrPheCysGluSerAspAlaLysLysCysIleAlaAlaAlaProHisMetGluAlaThrThrCysValLysProSerPhe
1690 1700 1710 1720 1730 1740 1750 1760 1770 1780 1790 1800
AACCAAGAGGACATTTACATCACCACAGAAAGCCTTACCACTACTGCCCGAATGTCCGAGACAGCAGATACCGCTCCAGATGCTGAGCCTGTCCCAGACTACACCACGGTTCACACCGTG
AsnGlnGluAspIleTyrIleThrThrGluSerLeuThrThrThrAlaArgMetSerGluThrAlaAspThrAlaProAspAlaGluProValProAspTyrThrThrValHisThrVal
1810 1820 1830 1840 1850 1860 1870 1880 1890 1900 1910 1920
AAGTCCCCAAGGGGCCTTATACTCAACGCGACTGCTTTGCCTTTGCCTGACAAAAAGAAGTTTCTCTCCTCATGTGGCTATGTGAGCACAGACCAACTGAACAAAATCATGCAGTAGCCT
LysSerProArgGlyLeuIleLeuAsnAlaThrAlaLeuProLeuProAspLysLysLysPheLeuSerSerCysGlyTyrValSerThrAspGlnLeuAsnLysIleMetGlnEnd
1930 1940 1950 1960 1970 1980 1990 2000 2010 2020 2030 2040
TTCCTATCTTTAATGGCAAAAGAAATGACTGGGCACGAACGCCTAAACCAAAACTATGTTTTAAATCTGTGTTGGGAGAGCGTGAGAGTGAATGTGGATTCAAAAATACCTTTTCTGGAA
2050 2060 2070 2080 2090 2100 2110 2120 2130 2140 2150 2160
ACGTCGAAACATTATCATAACGTGGAAAATCAAGAATATGTAATCAGATAGATACTCCCATTGTGAATTTTAAATATTTTAAAGAATCGTCTTTAAGACTGTTTAGTGGCAGTGATTGTC
2170 2180 2190 2200 2210 2220 2230 2240 2250 2260 2270 2280
TGTGCTGTGGGTCTTAACTTTGTGATACTAAGTATTAAATAGCTACGTTTTTCATGTATGTAGATCATGCTTTTTTGAAAAAGCAAAACAATCAGGTGGCTTTTGCAGTTCAGGAAATTG
2290 2300 2310 2320 2330 2340 2350 2360 2370 2380 2390 2400
AATGCAGATTTTAGCACAGACTGGATTTCTTTTTCCTTTTTAAATAGCTAGGAGCTAAAACTCTAGGGGAGAAGTAAAACTAGTTTGGATATGCAAAACACTTATTTTGACATAAAATTG
2410 2420 2430 2440 2450 2460 2470 2480 2490 2500 2510 2520
ATAAAGATATTTTTAATAATTTACACTGTAAGCATGAGTATTTTATAATACACTACAGATATATTGTAGTCCACAACAATCCATCTACGGATGTAACAACTACAATGTGTAGAGGGGTTG
2530
ATGCTTTGGTCAATCG

FIG.7

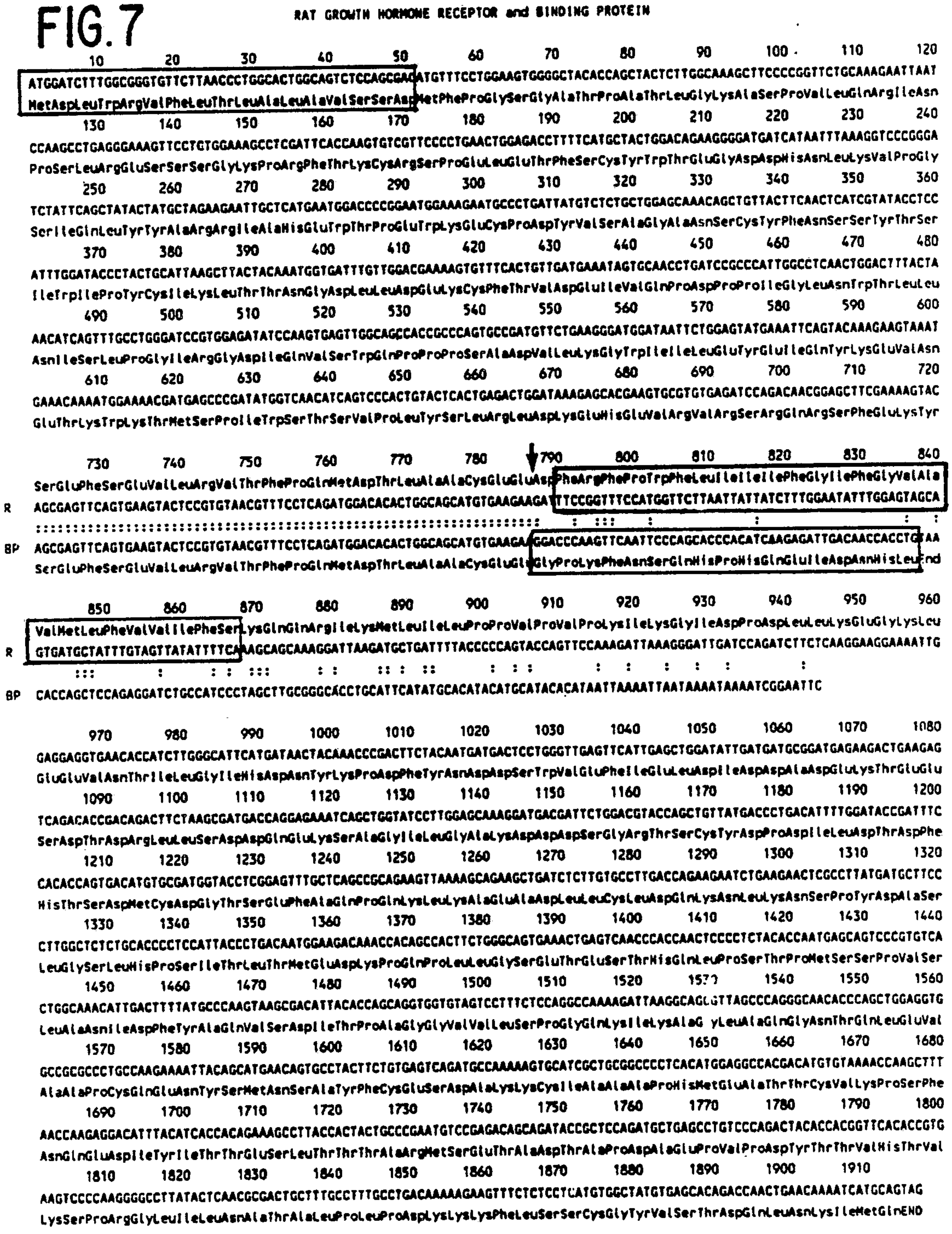

FIG.8

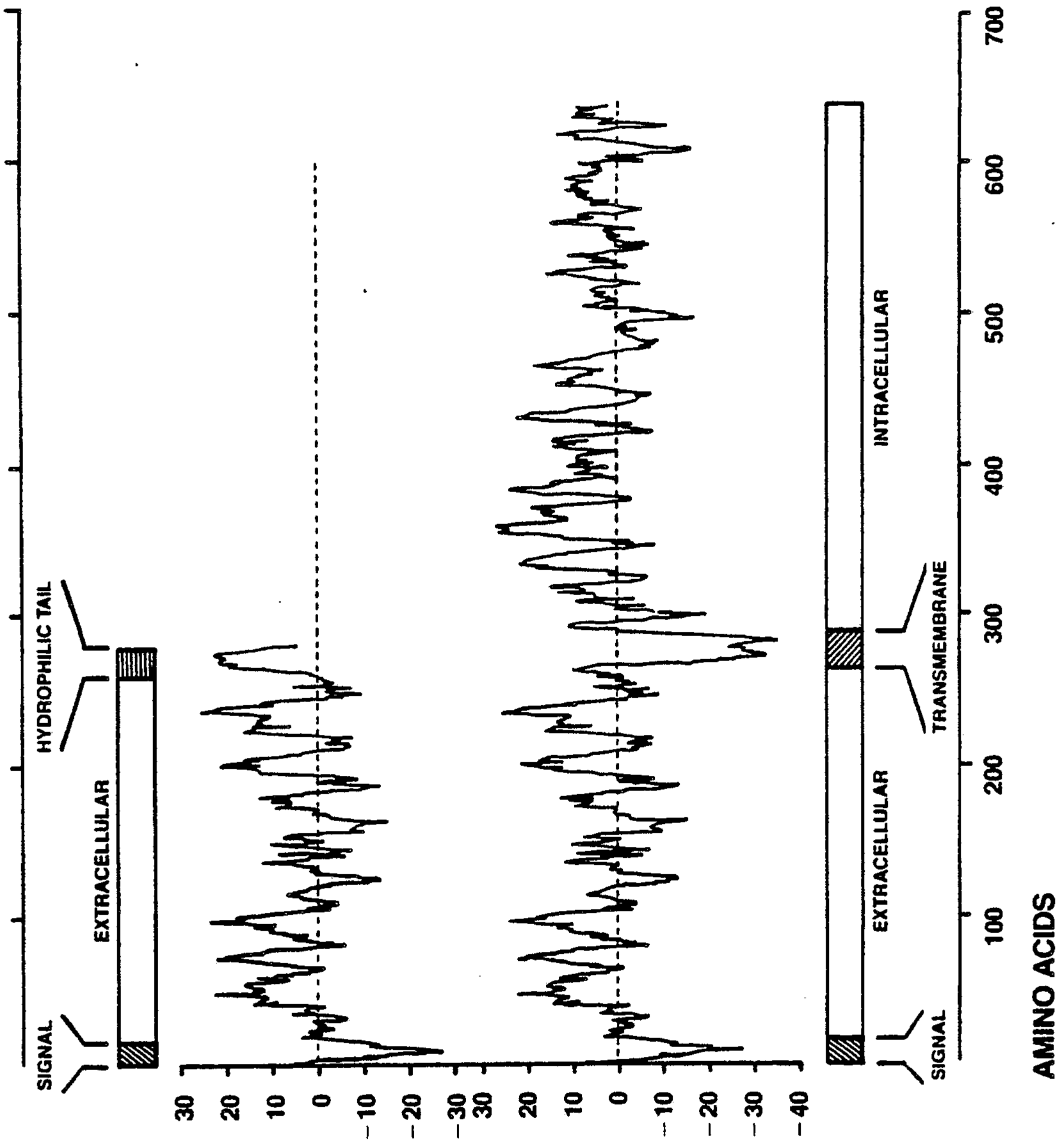
AMINO ACIDS
100
200
300
400
500
600
700
SIGNAL
EXTRACELLULAR
HYDROPHILIC TAIL
30
20
10
0
-10
-20
-30
30
20
10
0
-10
-20
-30
-40
EXTRACELLULAR
INTRACELLULAR
TRANSMEMBRANE
SIGNAL
AMINO ACIDS

FIG. 9

SOMATOTROPIN RECEPTOR and BINDING PROTEIN EXPRESSION

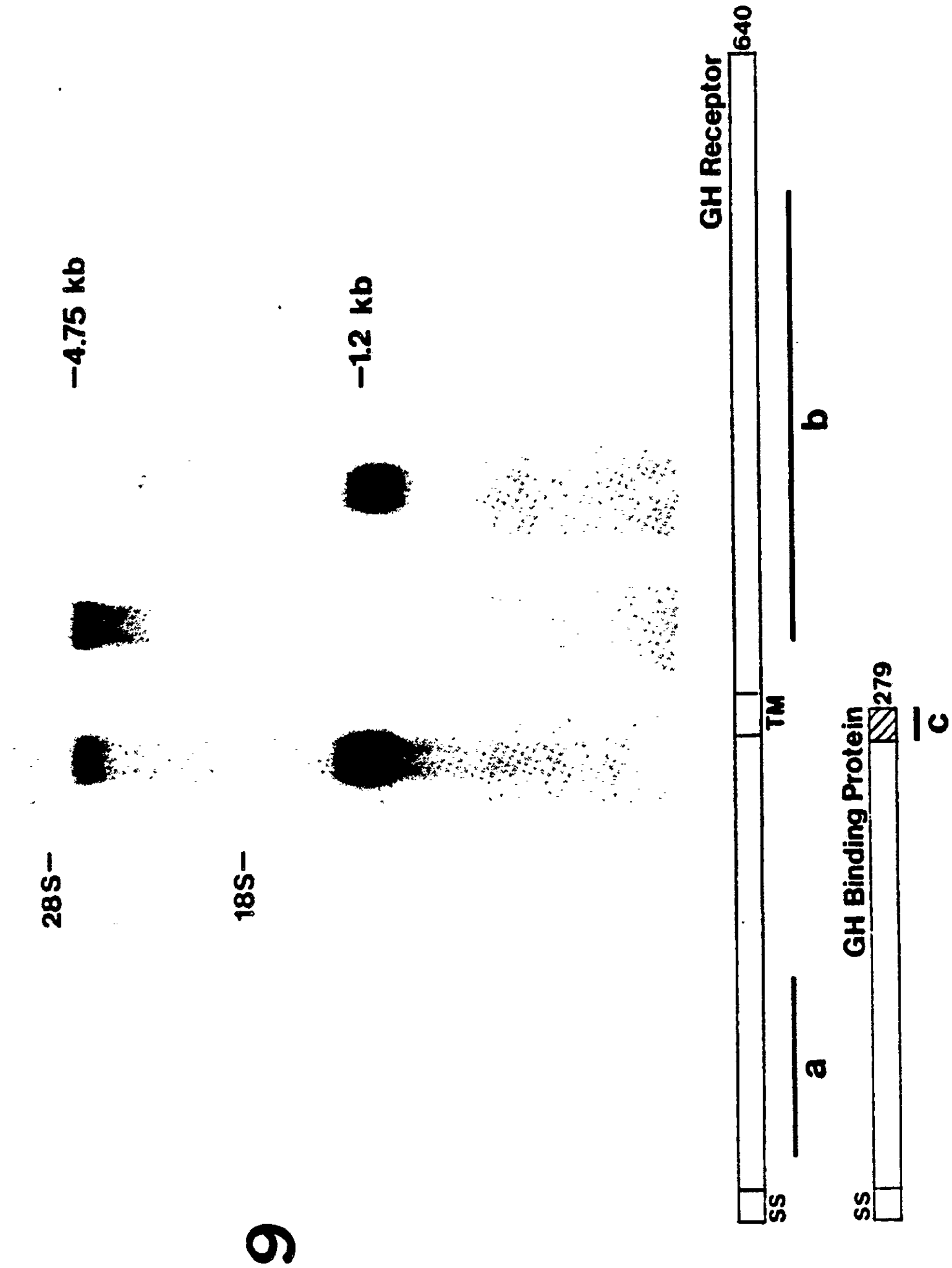

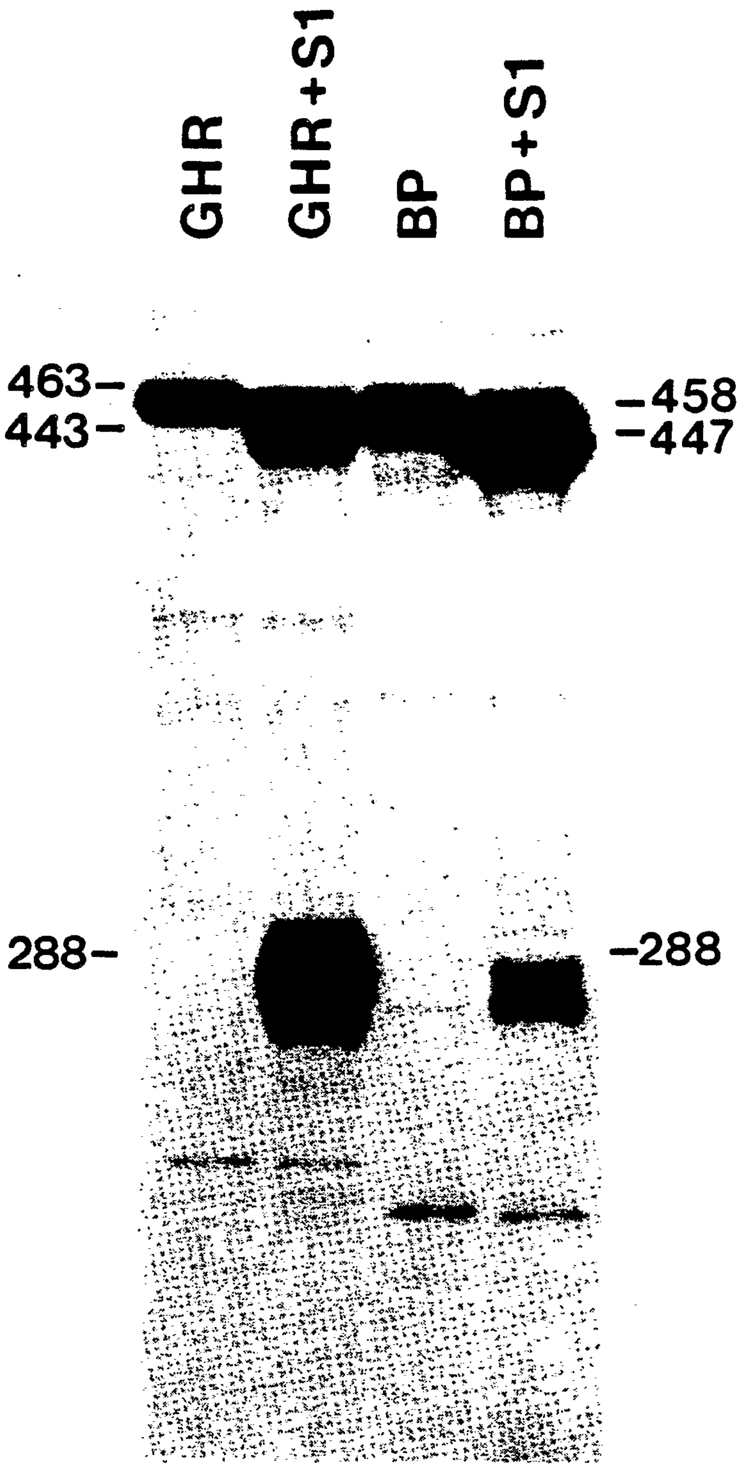


FIG. 10

FIG.11

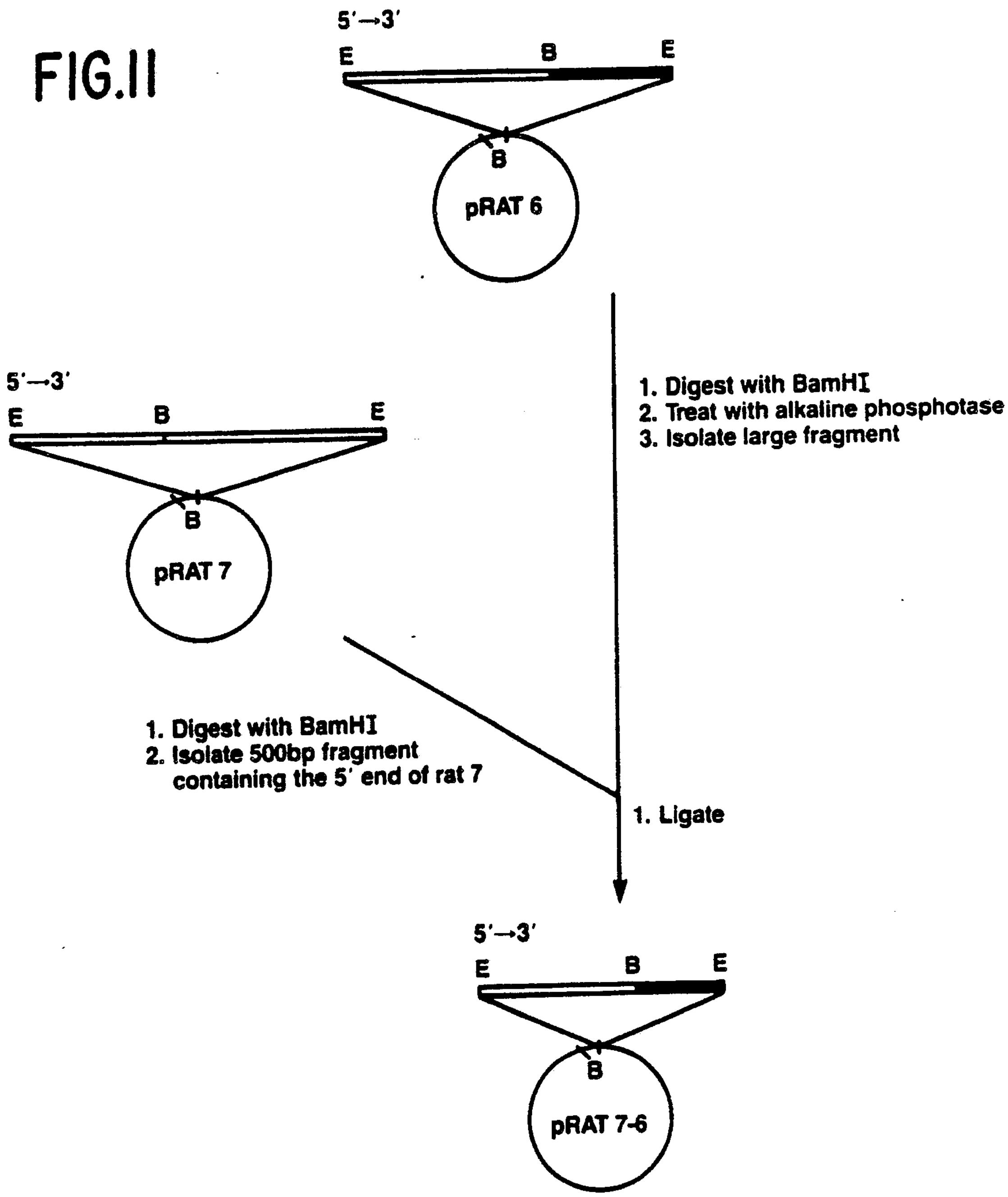
5'→3'
E
B
E
B
pRAT 6
1. Digest with BamHI
2. Treat with alkaline phosphotase
3. Isolate large fragment
5'→3'
E
B
E
B
pRAT 7
1. Digest with BamHI
2. Isolate 500bp fragment
containing the 5' end of rat 7
1. Ligate
5'→3'
E
B
E
B
pRAT 7-6

FIG.12

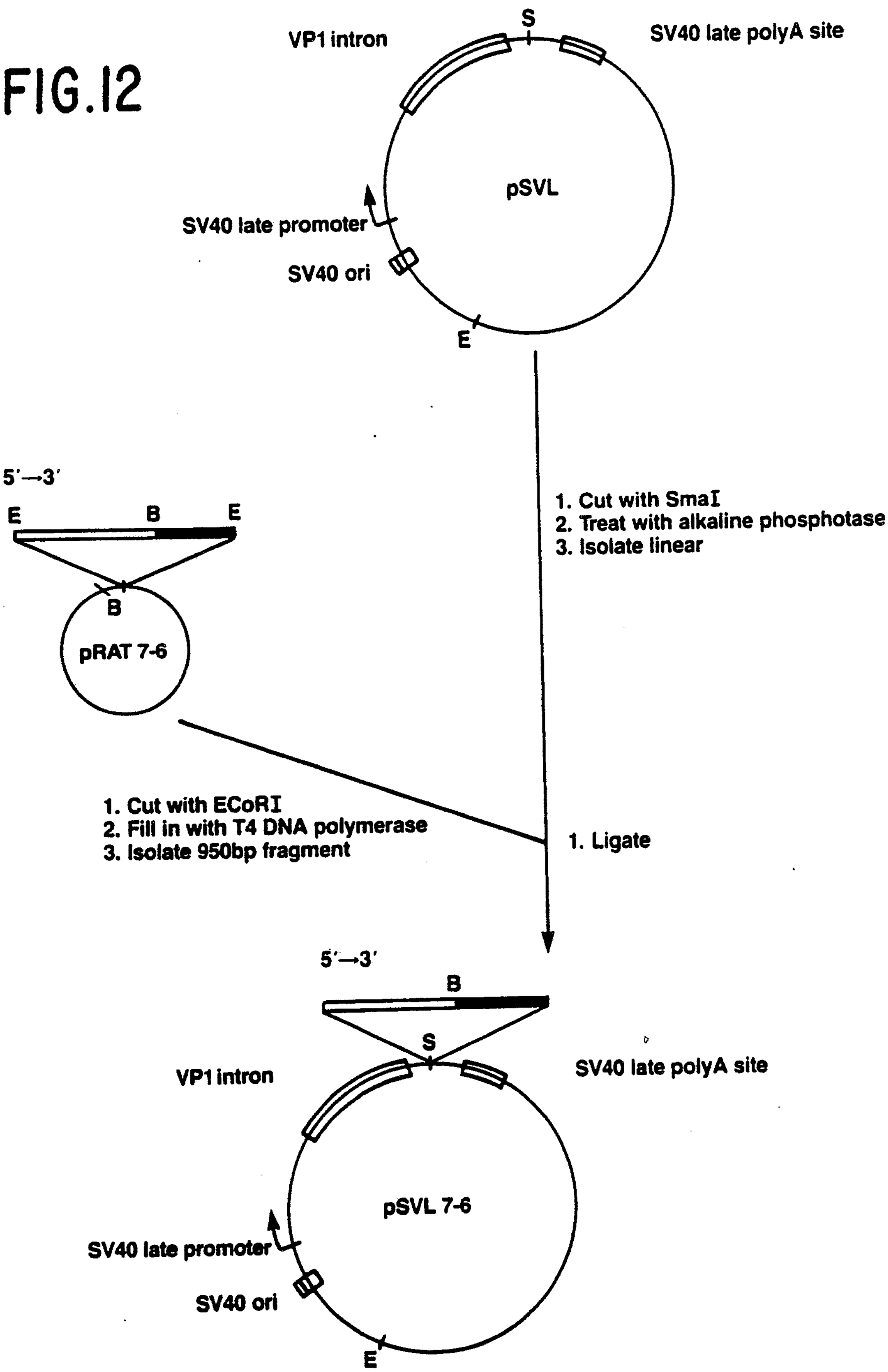
S
VP1 intron
SV40 late polyA site
pSVL
SV40 late promoter
SV40 ori
E
1. Cut with SmaI
2. Treat with alkaline phosphotase
3. Isolate linear
5'→3'
E
B
E
B
pRAT 7-6
1. Cut with ECoRI
2. Fill in with T4 DNA polymerase
3. Isolate 950bp fragment
1. Ligate
5'→3'
B
S
VP1 intron
SV40 late polyA site
pSVL 7-6
SV40 late promoter
SV40 ori
E

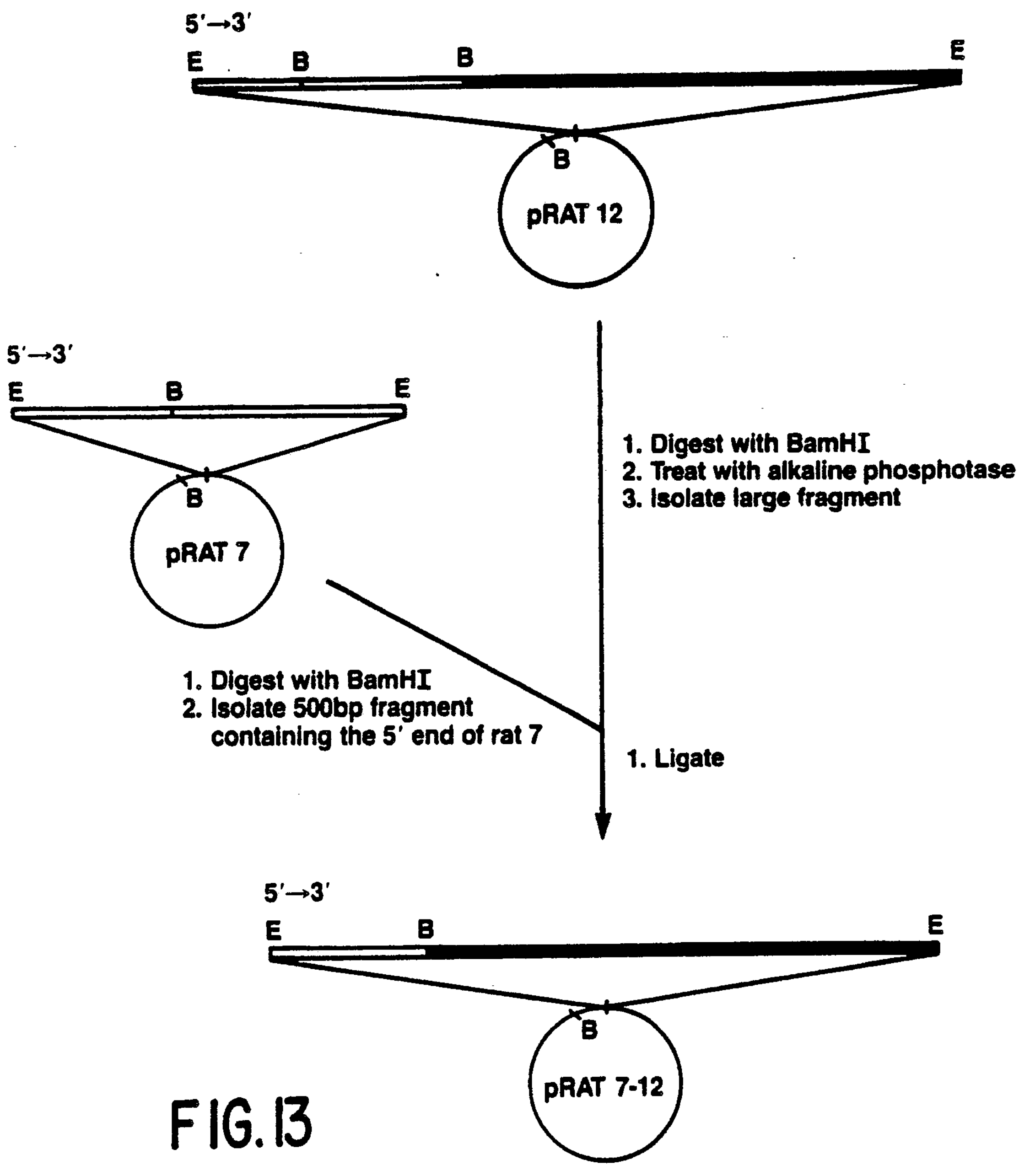
5'→3'
E
B
B
E
B
pRAT 12
5'→3'
E
B
E
B
pRAT 7
1. Digest with BamHI
2. Treat with alkaline phosphotase
3. Isolate large fragment
1. Digest with BamHI
2. Isolate 500bp fragment
containing the 5' end of rat 7
1. Ligate
5'→3'
E
B
E
B
pRAT 7-12

FIG. 13

FIG.14

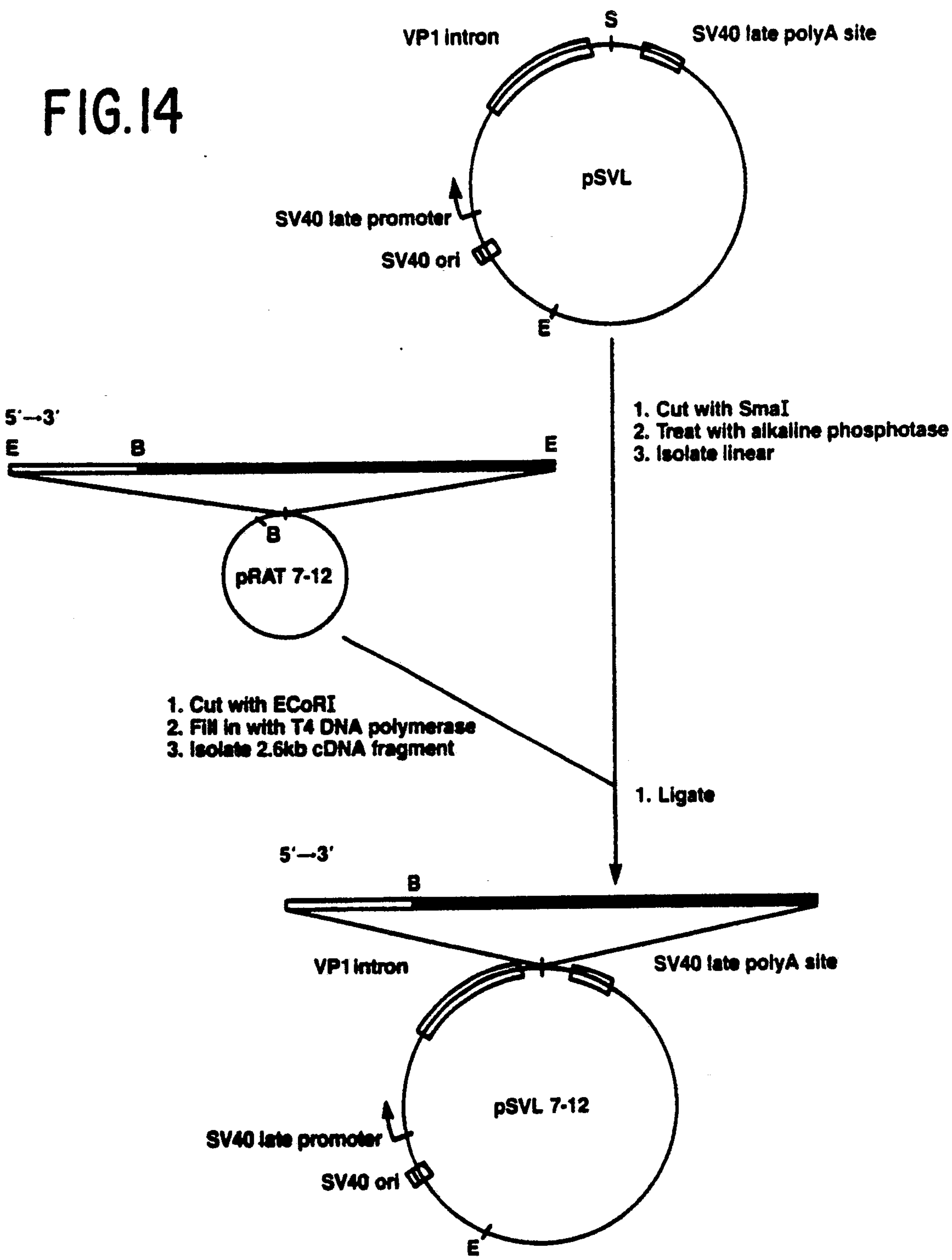
VP1 intron
S
SV40 late polyA site
pSVL
SV40 late promoter
SV40 ori
E
1. Cut with SmaI
2. Treat with alkaline phosphotase
3. Isolate linear
5'→3'
E
B
E
B
pRAT 7-12
1. Cut with ECoRI
2. Fill in with T4 DNA polymerase
3. Isolate 2.6kb cDNA fragment
1. Ligate
5'→3'
B
VP1 intron
SV40 late polyA site
pSVL 7-12
SV40 late promoter
SV40 ori
E

FIG. 15 Expressed Rat GH Receptor Competition Assay

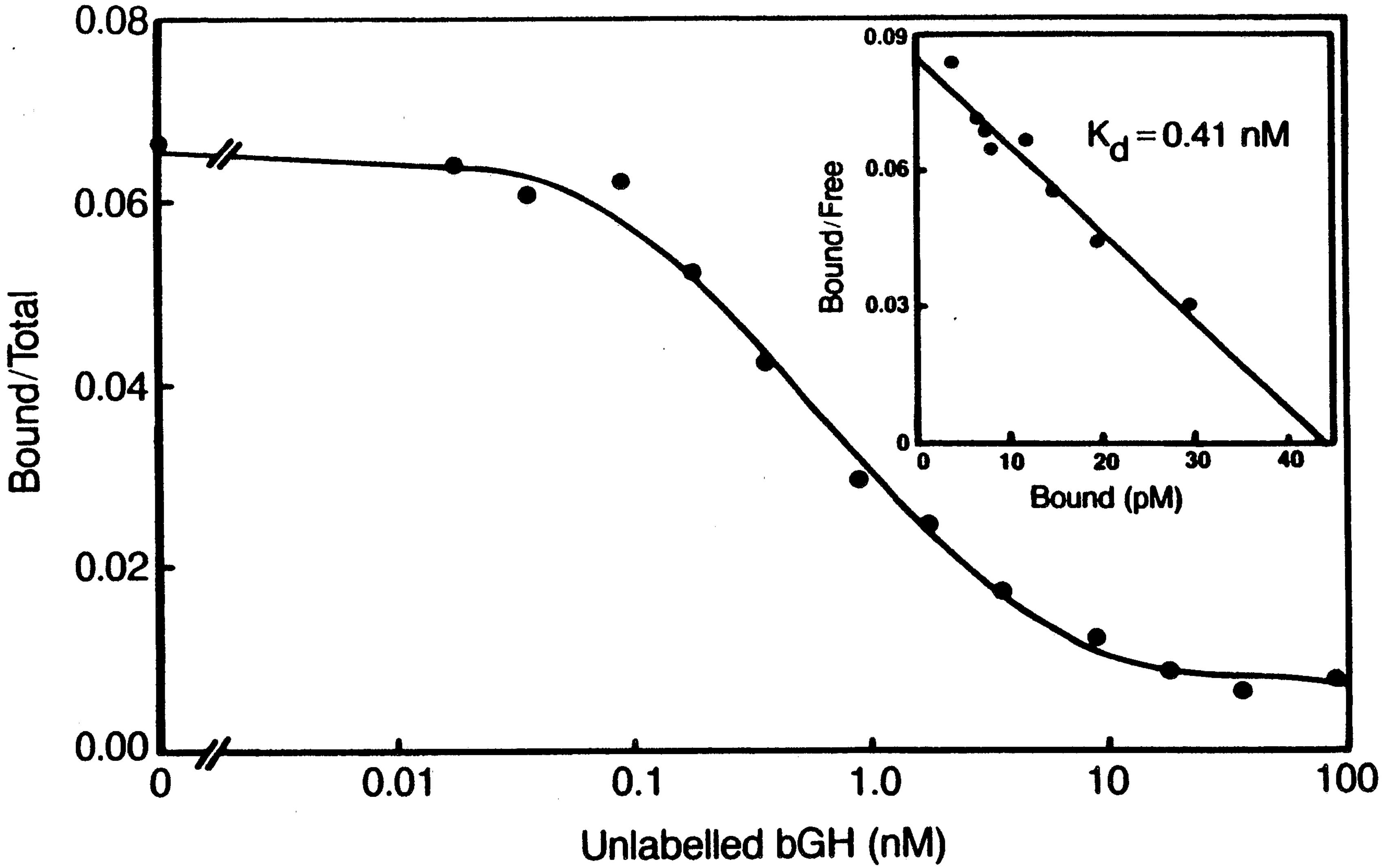

FIG. 16

Expressed Soluble Rat GH Binding Protein

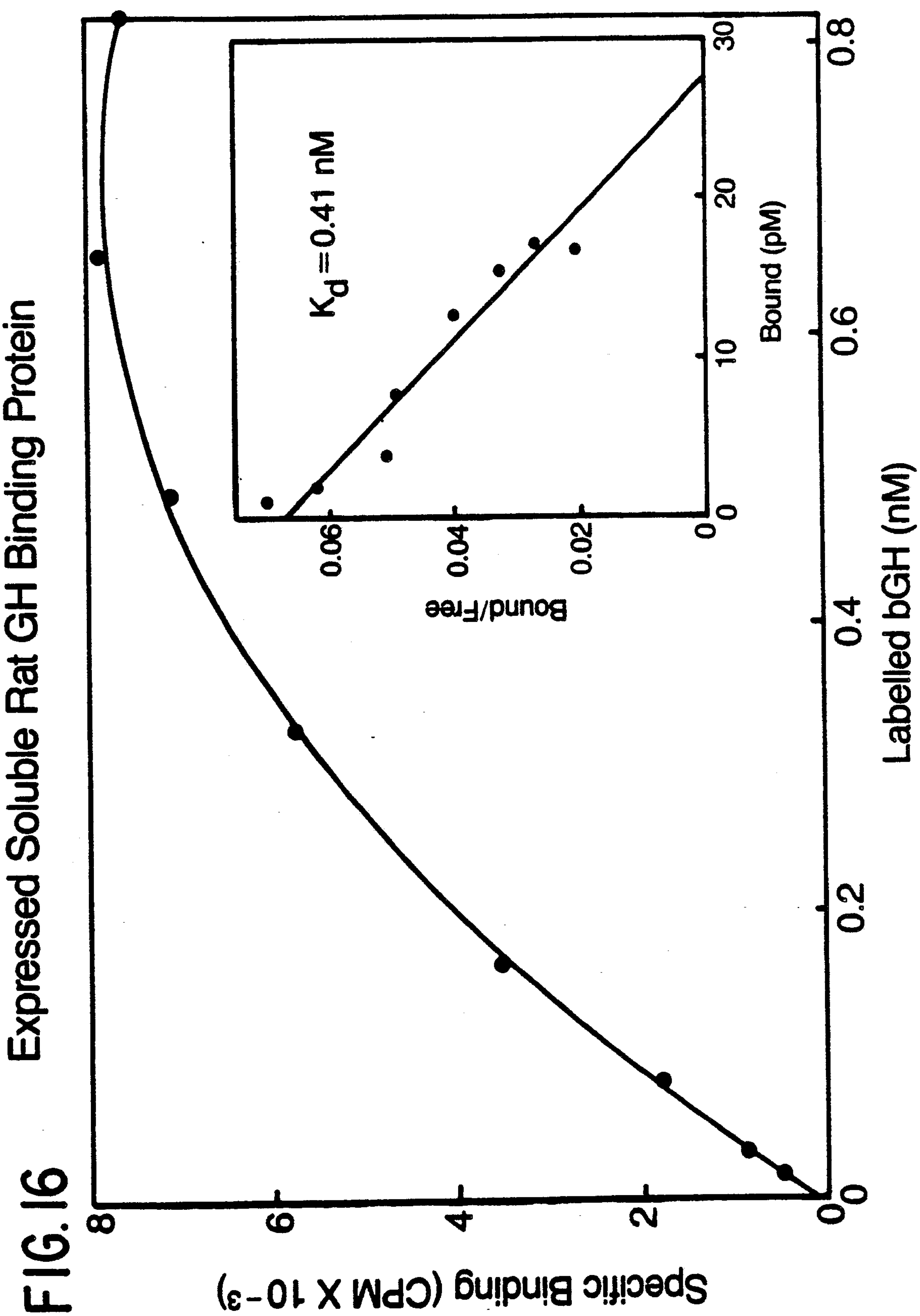

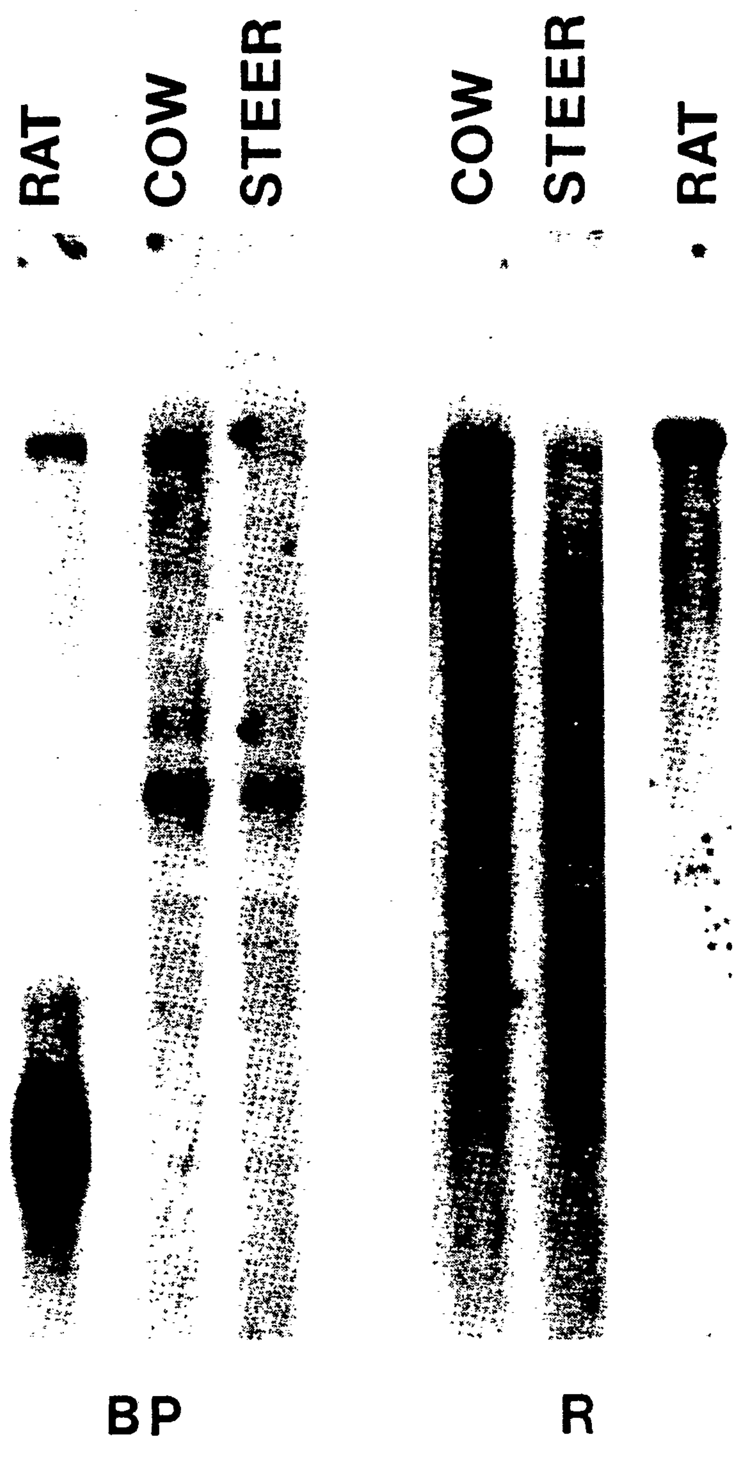


FIG. 17

```
         150                           160                           170
Rat   ProAspProProIleGlyLeuAsnTrpThrLeuLeuAsnIleSerLeuProGlyIleArgGlyAspIleGlnValSerTrpGlnProPro
Pig                                                   Thr      HisAla            Arg   Glu
Sheep             Val                                 ThrGlu   HisAla      Leu   Lys   Leu
Cow   ..........................................................................................

         180                           190                           200
      ProSerAlaAspValLeuLysGlyTrpIleIleLeuGluTyrGluIleGlnTyrLysGluValAsnGluThrLysTrpLysThrMetSer
         Asn         Gln            Val            Leu                           Gln      Met   Asp
         AsnThr      LysMet                        LeuHis         Leu            Gln      Met   Asp
      ..........................................................................................

         210                           220                           230
      ProIleTrpSerThrSerValProLeuTyrSerLeuArgLeuAspLysGluHisGluValArgValArgSerArgGlnArgSerPheGlu
         ValLeu               Val                        Tyr                              AsnSer
         LeuLeuVal            Met                        Tyr               Thr            AsnThr
      ..........................................................................................

         240                           250                           260
      LysTyrSerGluPheSerGluValLeuArgValThrPheProGlnMetAspThrLeuAlaAlaCysGluGluAspPheArgPheProTrp
            Gly                  Tyr      Leu         SerProPhe***
            GlyLys               LeuIle               AsnProSer***                  Gln
            GlyLys               LeuIle               AsnProSer***                  Gln

         270                           280                           290
      PheLeuIleIleIlePheGlyIlePheGlyValAlaValMetLeuPheValValIlePheSerLysGlnGlnArgIleLysMetLeuIle
                                    LeuThr   Ile      LeuLeu
                              Leu   LeuThr   Thr      LeuLeu
                              Val   Leu      Thr      LeuLeu

         300                           310                           320
      LeuProProValProValProLysIleLysGlyIleAspProAspLeuLeuLysGluGlyLysLeuGluGluValAsnThrIleLeuGly

                                                                                             Ala
                                                                                             Ala

         330                           340                           350
      IleHisAspAsnTyrLysProAspPheTyrAsnAspAspSerTrpValGluPheIleGluLeuAspIleAspAspAlaAspGluLysThr
                        HisGlu      Ser                                          Pro
                        HisGlu                                                   Pro
                        HisGlu                                                   Pro

         360                           370
      GluGluSerAspThrAspArgLeuLeuSerAspAspGlnGluLysSerAlaGlyIle
         Gly                     AsnAsn   His         LeuThr
         Gly                        Asn   His         LeuSer
         Gly                        Asn   His
```

Translation of cDNA clones of pig, sheep and cow growth hormone receptors. The amino acid sequences in the region of residues 149 to 377 of the rat protein are compared (cow is shown only from residue 239). Blank spaces represent identical amino acids. The predicted transmembrane domain of rat is from 266 to 288. Deletions in pig, sheep and cow sequences are denoted with asterisks.

**FIG.18**

FIG.19

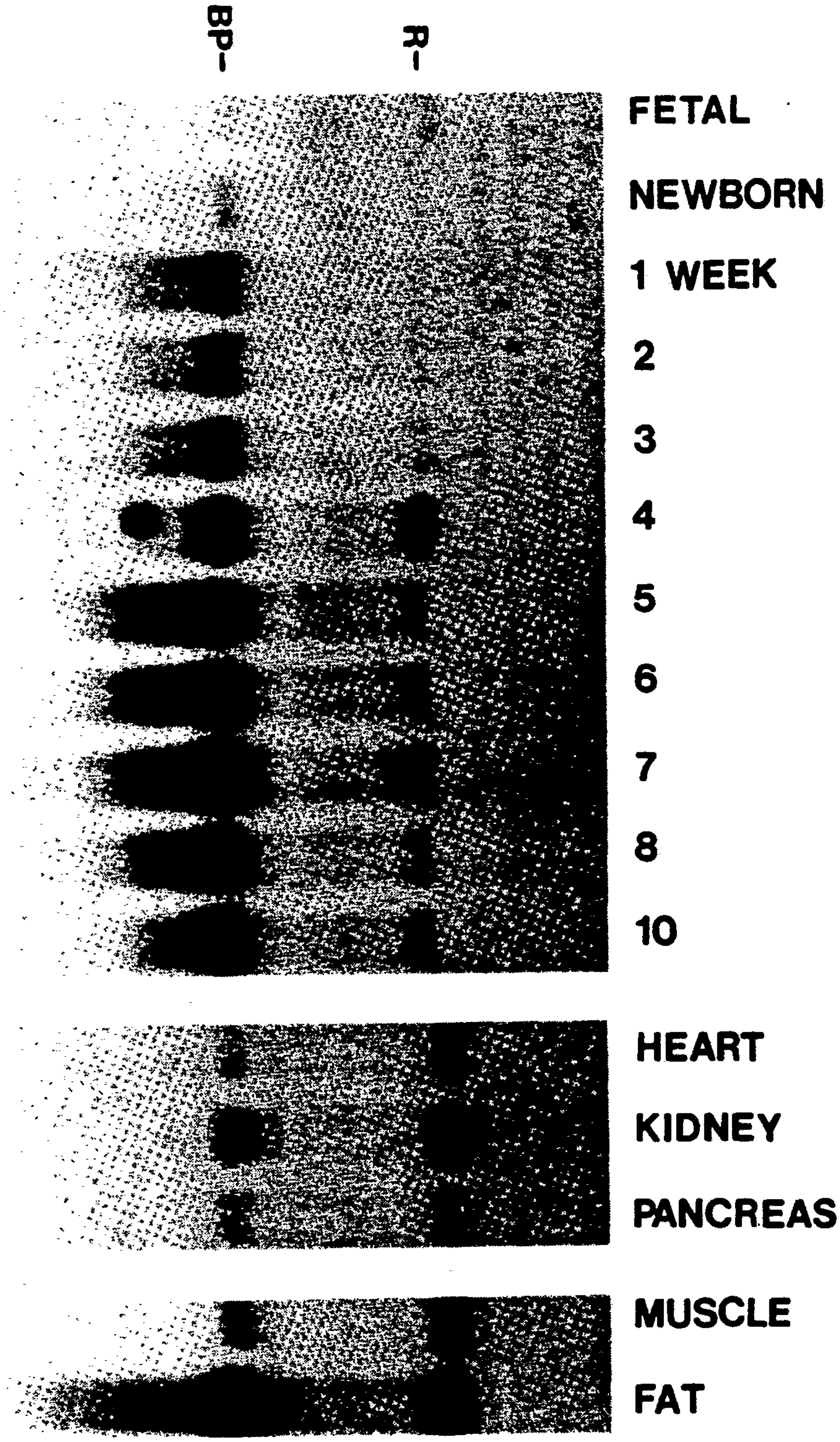
BP-
R-
FETAL
NEWBORN
1 WEEK
2
3
4
5
6
7
8
10
HEART
KIDNEY
PANCREAS
MUSCLE
FAT

FIG.20

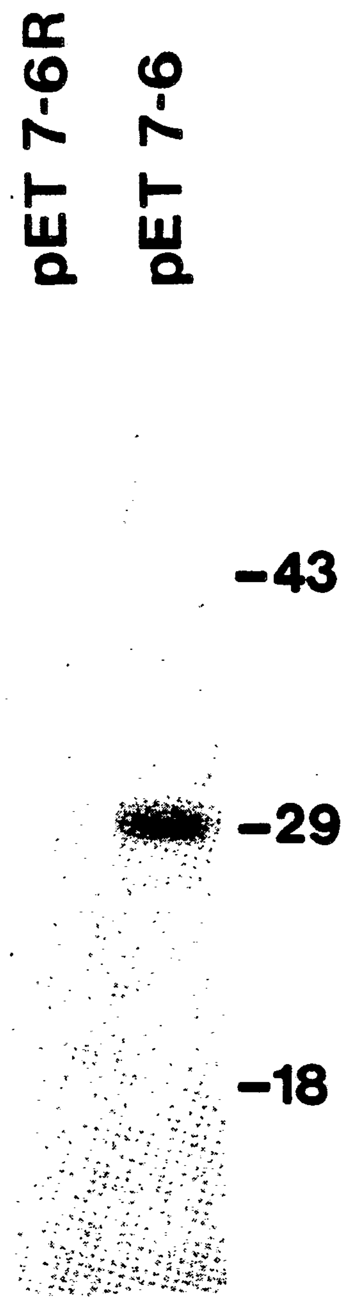

FIG. 21

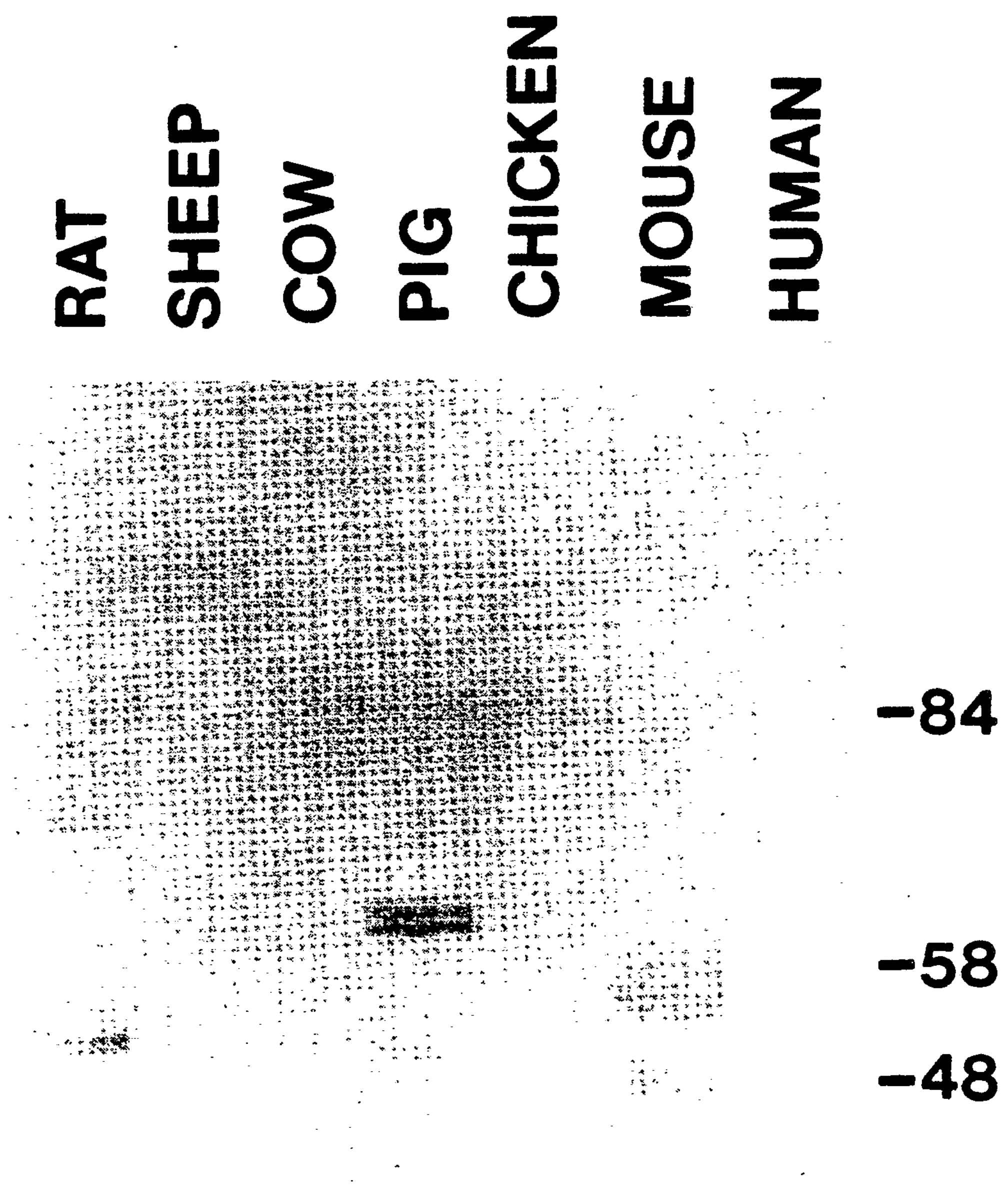

FIG. 22

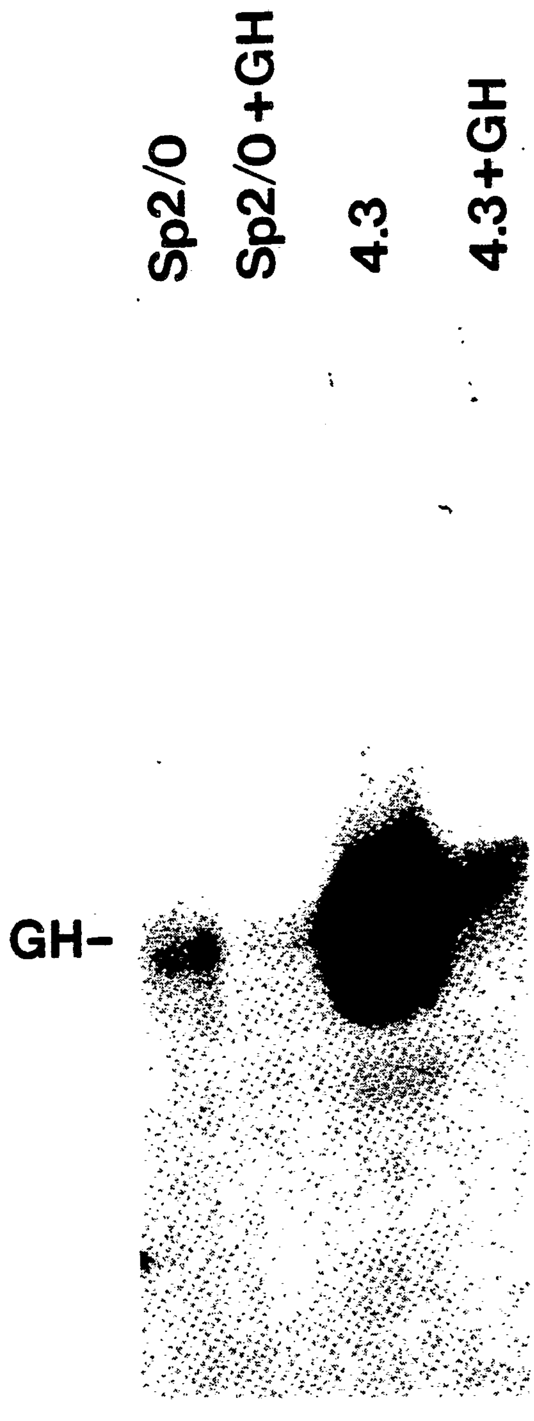

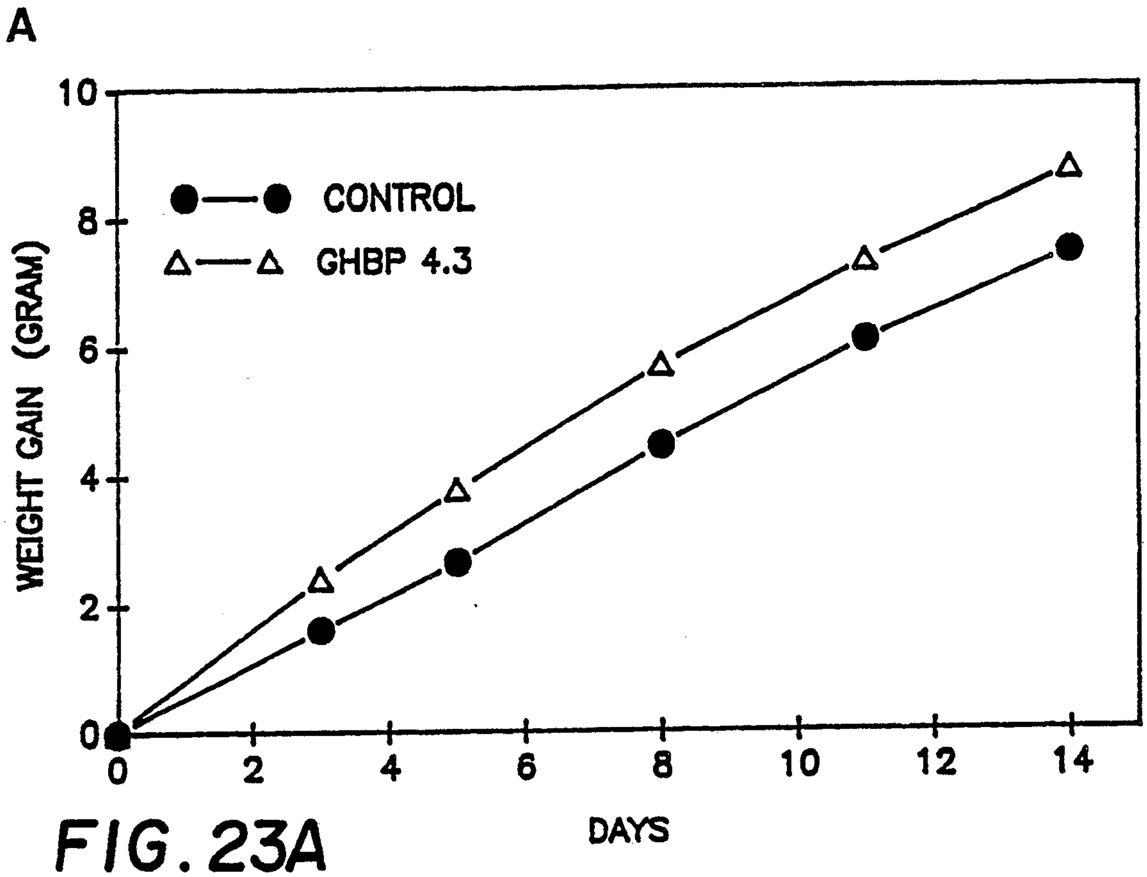


FIG. 23A

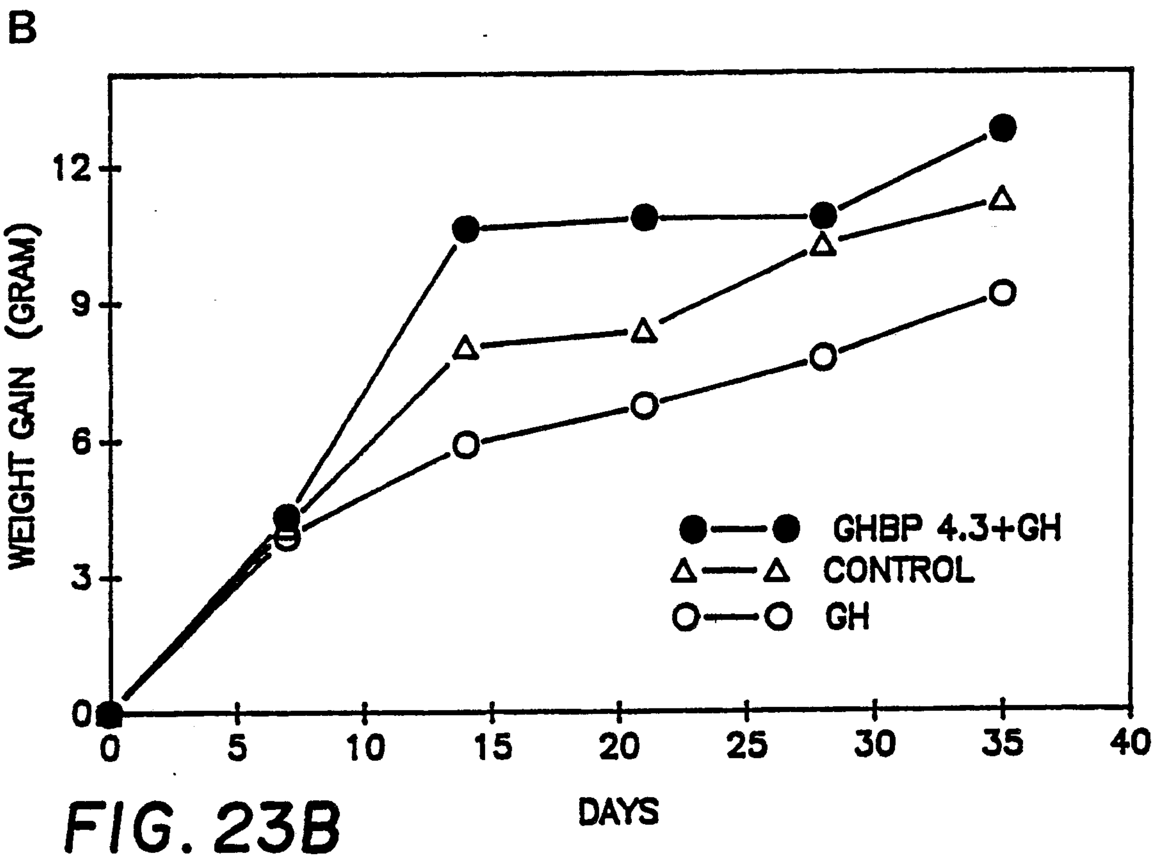


FIG. 23B

European Patent Office

# EUROPEAN SEARCH REPORT

Application Number

EP 90 10 2552

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 809 818 (GENENTECH INC.) * Page 41, lines 1-36; page 48, line 32 - page 50, line 7; page 55, line 34 - page 56, line 2; example 8 * --- | 1,2,4,5,6,7,9,10,11,12,13,14 | C 07 K 15/06<br>C 12 N 15/12<br>C 12 P 21/02<br>A 61 K 37/02<br>C 12 Q 1/68<br>A 61 K 37/36 //<br>(A 61 K 37/36<br>A 61 K 37:02 ) |
| X,D | NATURE, vol. 330, 10th December 1987, London UK, pages 537-543; D.W. LEUNG et al.: "Growth hormone receptor and serum binding protein: purification, cloning and expression" * Page 541, column 2, lines 44-59 * --- | 1,2,4-7,9-14 | |
| A | BIOCHEMICAL AND BIOPHYSICAL RESEARCH COMMUNICATIONS, vol. 139, no. 1, 29th August 1986, pages 150-155, Academic Press, Inc., Duluth, MN, US; A.C. HERINGTON et al.: "Affinity purification and structural characterization of a specific binding protein for human growth hormone in human serum" --- | 1 | **TECHNICAL FIELDS SEARCHED (Int. Cl.5)** |
| A | PROCEEDINGS OF THE NATIONAL ACADEMY OF SCIENCES, USA, vol. 85, December 1988, pages 9576-9579; W.C. SMITH et al.: "Detection of two growth hormone receptor mRNAs and primary translation products in the mouse" --- | 1 | C 12 N<br>C 12 P |
| P,X<br>D | MOLECULAR ENDOCRINOLOGY, vol. 3, no. 6, June 1989, pages 984-990, The Endocrine Society, Baltimore, Md, US; W.C. SMITH et al.: "Mouse serum growth hormone (GH) binding protein has GH receptor extracellular and substituted transmembrane domains" --- -/- | 1-14 | |

The present search report has been drawn up for all claims

| Place of search | Date of completion of the search | Examiner |
|---|---|---|
| THE HAGUE | 02-05-1990 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 90 10 2552

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | GENES & DEVELOPMENT, vol 3, 1989, pages 1199-1205, Cold Spring Harbor Laboratory Press, US; W.R. BAUMBACH et al.: "The growth hormone-binding protein in rat serum is an alternatively spliced form of the rat growth hormone receptor" ----- | 1-14 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 02-05-1990 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document